# EUROPEAN PATENT SPECIFICATION

(11) **EP 2 040 753 B1**
(45) Date of publication and mention of the grant of the patent: **27.02.2019**
(21) Application number: 07734620.3
(22) Date of filing: 21.05.2007
(51) Int. Cl.: A61K 45/00, A61K 39/00

(54) **PROGASTRIN INHIBITORS IN THE TREATMENT OF COLON CANCER**
PROGASTRIN INHIBITOREN ZUR BEHANDLUNG VON COLONKREBS
TRAITMENT DE TUMEUR INTESTINALE AVEC DES INHIBITEURES DE PROGASTRIN

(30) Priority: 22.05.2006 EP 06290823
(43) Date of publication of application: 01.04.2009
(73) Proprietor: INSERM (Institut National de la Santé et de la Recherche Médicale), 75654 Paris Cedex 13 (FR); CENTRE NATIONAL DE LA RECHERCHE SCIENTIFIQUE -CNRS-, 75794 Paris Cedex 16 (FR); Université de Montpellier, 34090 Montpellier (FR)
(72) Inventor: HOLLANDE, Frédéric, F-34880 Laverune (FR); JOUBERT, Dominique, F-34400 Saturargues (FR); JAY, Philippe, F-34980 Combaillaux (FR); PANNEQUIN, Julie, F-34200 Sète (FR); DELAUNAY, Nathalie, F-34090 Montpellier (FR); BOURGAUX, Jean-François, F-30980 Langlade (FR)
(74) Representative: Regimbeau
(86) International application number: PCT/IB2007/001312
(87) International publication number: WO 2007/135542

(56) References cited:
- WO-A-2004/089976
- WO-A-2006/032980
- LEPORCELET M ET AL: "Small-Molecule Antagonists of the Oncogenic Tcf/Beta-Catenin Protein Complex" CANCER CELL, US, vol. 5, no. 1, January 2004 (2004-01), pages 91-102, XP002984831 ISSN: 1535-6108
- SIDDHESHWAR R K ET AL: "Plasma levels of progastrin but not amidated gastrin or glycine extended gastrin are elevated in patients with colorectal carcinoma" GUT, vol. 48, no. 1, January 2001 (2001-01), pages 47-52, XP002398546 ISSN: 0017-5749
- KOYAMA TORU ET AL: "Mutation and expression of the beta-catenin-interacting protein ICAT in human colorectal tumors." JAPANESE JOURNAL OF CLINICAL ONCOLOGY, vol. 32, no. 9, September 2002 (2002-09), pages 358-362, XP002398547 ISSN: 0368-2811
- SINGH P ET AL: "INCOMPLETE PROCESSING OF PROGASTRIN EXPRESSED BY HUMAN COLON CANCER CELLS: ROLE OF NONCARBOXYAMIDATED GASTRINS" AMERICAN JOURNAL OF PHYSIOLOGY, AMERICAN PHYSIOLOGICAL SOCIETY, BETHESDA, MD, US, vol. 266, no. 3,PART 1, 1994, pages G459-G468, XP009057913 ISSN: 0002-9513
- P. D. OTTEWELL: "COOH-terminal 26-amino acid residues of progastrin are sufficient for stimulation of mitosis in murine colonic epithelium in vivo", AMERICAN JOURNAL OF PHYSIOLOGY: GASTROINTESTINAL AND LIVER PHYSIOLOGY, vol. 288, no. 3, 21 October 2004 (2004-10-21), pages G541-G549, XP055013222, ISSN: 0193-1857, DOI: 10.1152/ajpgi.00268.2004
- SINGH POMILA ET AL: "Mice overexpressing progastrin are predisposed for developing aberrant colonic crypt foci in response to AOM", 20000301, vol. 278, no. 3 part 1, 1 March 2000 (2000-03-01), pages G390-G399, XP002188103,

## Description

### FIELD OF THE INVENTION

The present invention relates to compositions for the treatment and the prevention of colorectal cancer, adenomatous polyposis and metastases.

### BACKGROUND OF THE INVENTION

Tumorigenesis of the human colon involves in 66 % of the cases somatic mutations of the tumor-suppressor gene adenomatous polyposis coli (APC) or in the beta-catenin gene (mean value calculated from the following list of references: (Conlin et al., 2005; De Filippo et al., 2002; Huang et al., 1996; Johnson et al., 2005; Kim et al., 2003; Luchtenborg et al., 2005; Mikami et al., 2006; Morin et al., 1997; Powell et al., 1992; Rowan et al., 2000; Segditsas and Tomlinson, 2006; Shitoh et al., 2004; Smith et al., 2002; Sparks et al., 1998; Suraweera et al., 2006; Takayama et al., 2001)). These mutations are considered as an early event of the colorectal carcinogenesis occurring in patients with sporadic colorectal cancer. Germinal mutations in the *apc* gene are also responsible for familial polyposis coli, a hereditary syndrome associated with a high risk of intestinal cancer. These mutations result in defectuous regulation of the cytoplasmic pool of the *adherens* junction protein beta-catenin, resulting in a constitutive activation of the Tcf-4-mediated transcriptional pathway. This constitutive activation results in a high level of transcription of Tcf4 target genes, such as c-myc or cyclin D1. Another potential target of this pathway is the GAST gene encoding the progastrin prohormone.
Transcription of this gene was shown to be increased after activation of the beta-catenin / Tcf4 complex *in vitro* (Koh et al., 2000).
A role for progastrin in colon carcinogenesis was first suggested for around 15 years ago, when progastrin was detected in colorectal tumor extracts (Finley et al., 1993; Kochman et al., 1992; Nemeth et al., 1993), and when plasma levels of progastrin were shown to be elevated in around 75 % of the patients bearing a colorectal tumor, while being undetectable in controls (Ciccotosto et al., 1995; Konturek et al., 2002; Siddheshwar et al., 2001; Van Solinge et al., 1993). Based on these observations, the theory that progastrin could be involved in the colon tumorigenesis has been investigated both *in vitro* and *in vivo.* Studies first started by showing that glycine-extended gastrin (one of the products of progastrin maturation) had a positive effect on proliferation (Hollande et al., 1997; Koh et al., 1999; Litvak et al., 1999; Singh et al., 1994; Stepan et al., 1999). Meanwhile, it was shown that overexpression of progastrin in the liver, where its maturation into smaller peptides cannot occur, induces mitogenesis in the intestinal epithelium (Wang et al., 1996). These same transgenic mice were used to demonstrate that progastrin overexpression induces a susceptibility to azoxymethane-induced carcinogenesis (Singh et al., 2000). However, this observation was challenged when it was shown that knocking-down the gastrin gene also induced the same susceptibility to carcinogenesis (Cobb et al., 2002). The proliferative effects of progastrin were however further demonstrated *in vitro* on the rat intestinal cell fine IEC6 (Brown et al., 2003), and *in vivo* on a transgenic mouse model displaying intestinal overexpression of progastrin under the control of the fabp promoter (Cobb et al., 2004). However, a major disadvantage of this mouse model is that progastrin is overexpressed in the differentiated cells of the intestine epithelium, and not in the proliferative ones. In 2003 and 2005, Ottewell et al provided data on the fact that progastrin stimulates murine colonic epithelial mitosis after DNA damage and on the fact that the COOH-terminal 26-amino acid residues of progastrin are sufficient for stimulation of mitosis in murine colonic epithelium *in vivo* (Ottewell et al., 2005; Ottewell et al., 2003).

Based on these data coming mostly from overexpression experiments, progastrin was accepted as a "growth factor" on intestinal epithelial cells. In contrast, only three studies have tried to deplete cells *in vitro* and one *in vivo. In vitro,* 2 progastrin-producing cell lines were transfected with a gastrin gene antisense and it was shown that colony forming was diminished as well as tumor engrafting in nude mice (Singh et al., 1996). Importantly however, it was not demonstrated to which gastrin gene product these blocking effects were attributed. A more recent study demonstrated that progastrin depletion allowed the restoration of stable adherens and tight junctions in intestinal epithelial cells, linking progastrin with the activation of migration (Hollande et al., 2003). Finally, the last *in vitro* study was focused on the role of glycine-extended gastrin 17, a maturation product of progastrin, since the cells used in that work secrete large amounts of this peptide but very little progastrin (Hollande et al., 1997). *In vivo,* the KO mice, as mentioned above, were more susceptible to tumorigenesis.

Importantly, the only two studies reporting a connection between the beta-catenin / Tcf4 complex and the gastrin family of peptides demonstrated *in vitro* only that activation of the Tcf4 transcriptional pathway (Koh et al., 2000), particularly in correlation with Ras (Chakladar et al., 2005), leads to activation of the gastrin gene promoter and to increased transcription of the gene. In contrast, prior to the data provided here, nothing was known concerning the actual nature of the gastrin gene-derived peptide secreted in the colon upon abnormal activation of the beta-catenin / Tcf4 pathway, no report mentioned the possibility to reverse the activation of this pathway through a blockage of progastrin function, and no correlation had been established in human colorectal cancer patients between those displaying an activation of this pathway within their tumor and those who have elevated expression of the gastrin gene. Consequently, it appears that no prior data provided the sufficient scientific evidence to show that depletion of progastrin could reverse colorectal tumorigenesis induced by a constitutive beta-catenin / Tcf4 activation. Such data are provided below.

### Gastrin and Progastrin

Gastrin is a classical gut peptide hormone, which was identified originally as a stimulant of gastric acid secretion. It is produced principally by the G cells of the Gastric antrum and to a variable extent in the upper small intestine, with much lower amounts in the colon and pancreas. Over recent years, there has been increasing interest in the role of the gastrin family of peptides in colorectal carcinogenesis. In particular, evidence is accumulating that the precursor forms of gastrin (progastrin and glycine-extended gastrin), which were previously thought to be inactive, play a role in the development of colorectal cancer (see above comments). Gastrin occurs in various molecular forms. The human COOH terminally amidated gastrins, G17 and G34, are generated from a 101-amino acid precursor molecule, preprogastrin, by posttranslational modifications. Preprogastrin is cotranslationally translocated into the endoplasmic reticulum where the signal peptide is rapidly cleaved to give rise to progastrin. Progastrin is subsequently cleaved by prohormone convertases and carboxypeptidase E to give rise to a peptide with a COOH-terminal glycine residue, namely G34-Gly, and an additional cleavage generates the peptide G17-Gly. G34-Gly can be converted to the COOH terminally amidated peptide G34 by peptidyl alpha-amidating monooxygenase and can similarly be cleaved to generate G17. G17 or Gamide is the predominant antral form of gastrin, with nonamidated precursors (progastrin, glycine-extended gastrin) generally comprising less than 10 % of the total secreted peptide in humans. However, in certain clinical situations where processing is impaired, a greater proportion of nonamidated gastrin is secreted. For example, tissue and plasma concentrations of progastrin are elevated in some patients with colorectal carcinoma as mentioned above (Ciccotosto et al., 1995; Konturek et al., 2002; Siddheshwar et al., 2001; Van Solinge et al., 1993).

In WO 99/19353, a method of treatment of a condition associated with hyperactivity of autocrine stimulation of proliferation of cells of the gastrointestinal mucosa is suggested. The method comprises the step of administering an effective amount of an antagonist of binding of progastrin to intracellular gastrin/CCK-C receptors or other progastrin receptors to a mammal in need of such treatment.

US 6,165,990 discloses methods for the treatment of colon cancer. The expression of gastrin by colon cancers is inhibited by the use of antisense gastrin expression.

In WO 2004/089976 A, methods and compositions for the treatment of conditions associated with elevated levels of non-amidated gastrin are disclosed. In one aspect the method comprises the step of administering, to a mammal in need of such treatment, an effective amount of a compound which has the ability to inhibit the binding of ferric ions to any one or more of glycine-extended gastrin17, progastrin or progastrin-derived peptides, but which does not inhibit the activity of amidated gastrin, and thereby to inhibit the activity of non-amidated gastrins. Data is provided to support the claim that the capacity to inhibit the binding of ferric ions to glycine-extended gastrin17 does reduce its biological activity, but no data is provided concerning the effects on the biological activity of progastrin.

WO 2006/032980 A aims to protect progastrin-binding molecules that selectively binds progastrin, wherein the molecule does not bind gastrin-17 (G17), gastrin-34 (G34), glycine-extended gastrin-17 (G17-Gly), or glycine-extended gastrin-34 (G34-Gly), in particularly monoclonal antibodies selective for progastrin and hybridomas that produce them. A method is disclosed to quantify progastrin levels in biological fluids using the antibodies mentioned above, but this claim is not supported by any data concerning the ability of these antibodies to selectively detect progastrin in any biological fluid. A method of prevention or treatment of a gastrin-promoted disease or condition comprising the administration of antibodies is also disclosed, without data to support the claim that these antibodies demonstrate any selective ability to alter the biological activity of progastrin. This document provides only methods for establishing the specificity of such antibodies.

To date, effective and specific ways of treating and/or preventing colorectal cancer, metastases and adenomatous polyposis are scarce. Therefore, an improved method for treating and/or preventing colorectal cancer, metastasis or adenomatous polyposis is herein provided.

### SUMMARY OF THE INVENTION

The invention is defined in the appended claims and any other aspects or embodiments set forth herein are for information only.

Provided herein is a method for treating and/or preventing colorectal cancer, adenomatous polyposis or metastasis stemming from progastrin-secreting cells, in which the beta-catenin/Tcf-4-mediated transcriptional pathway is constitutively active, comprising the step of administering an effective amount of an inhibitor of progastrin-induced repression of the Inhibitor of beta-catenin and Tcf-4 binding (ICAT) to an individual in need thereof.

A method for treating and/or preventing colorectal cancer, adenomatous polyposis or metastasis displaying progastrin-secreting cells and cells in which the beta-catenin/Tcf-4-mediated transcriptional pathway is constitutively active, is also provided, said method comprising the step of administering an effective amount of an inhibitor of progastrin-induced repression of the Inhibitor of beta-catenin and Tcf-4 binding (ICAT) to an individual in need thereof.

It is herein described for the very first time that depletion of progastrin, and not any of the other products of the *GAST* gene, is able to reverse tumorigenesis by acting directly on the constitutive activation of the beta-catenin/Tcf4 activity. This reversal involves the regulation of ICAT expression, low in a context of high progastrin, high in the context of progastrin depletion. When ICAT is high, the constitutive activation of beta-catenin/Tcf4 activity is profoundly decreased due to the fact that ICAT hijacks beta-catenin away from Tcf4.

Therefore, for the first time, it is demonstrated that depletion of progastrin is a very efficient therapy for colorectal cancer, adenomatous polyposis or metastasis stemming from progastrin secreting cells, in which the beta-catenin/Tcf-4-mediated transcriptional pathway is constitutively active and for colorectal cancer, adenomatous polyposis or metastasis displaying progastrin-secreting cells and cells in which the beta-catenin/Tcf-4-mediated transcriptional pathway is constitutively active. The population of those human colorectal tumors, adenomatous polyposis or metastasis having a low ICAT expression, a constitutive beta-catenin/Tcf-4-mediated activity, and a high progastrin expression can thus be selected for anti-progastrin therapy.

A method for determining if a patient suffering from colorectal cancer, adenomatous polyposis or metastasis is responsive to a therapeutic treatment with an inhibitor of progastrin-induced repression of the Inhibitor of beta-catenin and Tcf-4 binding (ICAT) is also provided, said method comprising the step of determining whether the colorectal cancer, adenomatous polyposis or metastasis stems from progastrin secreting cells in which the beta-catenin/Tcf-4-mediated transcriptional pathway is constitutively active.

A method which combines the selection of a specific population of patients that are likely to be responsive to the treatment regimen, with the treatment and/or prevention of colorectal cancer, adenomatous polyposis or metastasis, is also provided.

Also provided is the use of an inhibitor of progastrin-induced repression of the Inhibitor of beta-catenin and Tcf-4 binding (ICAT) in the manufacture of a medicament for treating and/or preventing colorectal cancer, adenomatous polyposis or metastasis stemming from progastrin secreting cells, in which the beta-catenin/Tcf-4-mediated transcriptional pathway is constitutively active.

Also provided is the use of an inhibitor of progastrin-induced repression of the Inhibitor of beta-catenin and Tcf-4 binding (ICAT) in the manufacture of a medicament for treating and/or preventing colorectal cancer, adenomatous polyposis or metastasis displaying progastrin-secreting cells and cells in which the beta-catenin/Tcf-4-mediated transcriptional pathway is constitutively active.

It is also provided herein a method for screening for compounds for treating and/or preventing colorectal cancer, adenomatous polyposis or metastasis stemming from progastrin secreting cells, in which the beta-catenin/Tcf-4-mediated transcriptional pathway is constitutively active.

### DETAILED DESCRIPTION OF THE INVENTION

A method for treating and/or preventing colorectal cancer, adenomatous polyposis or metastasis displaying progastrin-secreting cells and cells in which the beta-catenin/Tcf-4-mediated transcriptional pathway is constitutively active, is provided, said method comprising the step of administering an effective amount of an inhibitor of progastrin-induced repression of the Inhibitor of beta-catenin and Tcf-4 binding (ICAT) to an individual in need thereof.

Also provided is the use of an inhibitor of progastrin-induced repression of the Inhibitor of beta-catenin and Tcf-4 binding (ICAT) in the manufacture of a medicament for treating and/or preventing colorectal cancer, adenomatous polyposis or metastasis displaying progastrin-secreting cells and cells in which the beta-catenin/Tcf-4-mediated transcriptional pathway is constitutively active.

Typically the progastrin secreting cells and the cells in which the beta-catenin/Tcf-4-mediated transcriptional pathway are colonic cells.

Typically progastrin-secreting cells and cells in which the beta-catenin/Tcf-4-mediated transcriptional pathway is constitutively active may be the same.

A method for treating and/or preventing colorectal cancer, adenomatous polyposis or metastasis stemming from progastrin secreting cells, in which the beta-catenin/Tcf-4-mediated transcriptional pathway is constitutively active, is also provided, said method comprising the step of administering an effective amount of an inhibitor of progastrin-induced repression of the Inhibitor of beta-catenin and Tcf-4 binding (ICAT) to an individual in need thereof.

Typically the progastrin secreting cells are colonic cells.

Also provided is the use of an inhibitor of progastrin-induced repression of the Inhibitor of beta-catenin and Tcf-4 binding (ICAT) in the manufacture of a medicament for treating and/or preventing colorectal cancer, adenomatous polyposis or metastasis stemming from progastrin secreting cells, in which the beta-catenin/Tcf-4-mediated transcriptional pathway is constitutively active.

Metastases from colorectal cancer are an important cause of death for patients, and they are rarely operated, either because they are difficult to access or because surgical removal would present critical risks for the immediate patient survival. This is notably the case when the metastatic mass has grown in close proximity to a vital artery. The treatment disclosed hereininduces a regression of these metastases and is therefore extremely useful, either by completely ridding the body of the metastases or at least by allowing surgical removal by forcing the metastases to shrink away from the artery. Furthermore down-regulation of progastrin synthesis in colorectal cancer cells restores the adhesive capacity of colorectal tumor cells and leads to a reduction of their migration potential (Hollande et al., 2003). Typically the metastases treated may not be surgically removable. The metastasis may originate from a primary tumor for which the activation status of the beta-catenin/Tcf-4-mediated transcriptional pathway is unknown. Typically the primary tumor from which the metastasis originates is a colon tumor.

By individual, it is meant animal or human being.

By constitutively active beta-catenin/Tcf-4-mediated transcriptional pathway, it is meant a permanent, and unregulated, activation of the pathway leading to the formation of a transcriptional complex between beta-catenin and Tcf-4, leading to an enhanced transcription of Tcf-4 target genes due to a lack of degradation of cytoplasmic beta-catenin.

Examples of cells in which the beta-catenin/Tcf-4-mediated transcriptional pathway is constitutively active, are cells in which the adenomatous polyposis coli (APC) tumor-suppressor gene is mutated or cells in which the beta-catenin gene is mutated in a way which prevents a normal degradation of beta-catenin.

Typically, before applying a method of treatment as disclosed herein to a patient suffering from colorectal cancer, adenomatous polyposis or metastasis, a diagnostic test may be performed in order to determine whether the colorectal cancer, adenomatous polyposis or metastasis stems from progastrin secreting cells in which the beta-catenin/Tcf-4-mediated transcriptional pathway is constitutively active or whether the colorectal cancer, adenomatous polyposis or metastasis displays progastrin-secreting cells and cells in which the beta-catenin/Tcf-4-mediated transcriptional pathway is constitutively active. By performing such a pre-treatment diagnostic test, it is possible to determine whether a patient would be responsive to a method of treatment disclosed herein.

It falls within the ability of the skilled artisan to carry out such a diagnostic test.

Typically, plasma levels of progastrin in the blood of the patients may be determined in order to treat only those patients having an elevated progastrin level in the plasma. Plasma progastrin survey may also be used to detect an eventual recurrence of the tumor and/or of metastases. Throughout their life, colorectal tumors secrete factors promoting the genesis of new blood vessels (such as VEGF), thereby ensuring that the tumor receives a sufficient amount of nutrients to achieve maximal growth (Wray et al 2004). The presence of these new vessels also allows the tumor to release various factors into the blood stream, and this is notably the case for progastrin. Progastrin-targeted therapy may therefore include the measurement of plasma progastrin levels.

Alternatively or additionally, mutations of the APC gene or of the beta-catenin gene may be detected or the abnormal level of transcription of Tcf target genes, such as for example c-Myc and cyclin D1, may be determined by staining for these markers on tissue slides taken from the patient or by using microarray technologies for example.

A method for determining if a patient suffering from colorectal cancer, adenomatous polyposis or metastasis is responsive to a therapeutic treatment with an inhibitor of progastrin-induced repression of the Inhibitor of beta-catenin and Tcf-4 binding (ICAT) is also provided, said method comprising the step of determining whether the colorectal cancer, adenomatous polyposis or metastasis stems from progastrin secreting cells in which the beta-catenin/Tcf-4-mediated transcriptional pathway is constitutively active or whether the colorectal cancer, adenomatous polyposis or metastasis displays progastrin-secreting cells and cells in which the beta-catenin/Tcf-4-mediated transcriptional pathway is constitutively active.

Typically the step of determining whether the colorectal cancer, adenomatous polyposis or metastasis stems from progastrin secreting cells in which the beta-catenin/Tcf-4-mediated transcriptional pathway is constitutively active or whether the colorectal cancer, adenomatous polyposis or metastasis displays progastrin-secreting cells and cells in which the beta-catenin/Tcf-4-mediated transcriptional pathway is constitutively active, comprises the step of measuring plasma levels of progastrin of said patient. Additionally or alternatively the step of determining whether the colorectal cancer, adenomatous polyposis or metastasis stems from progastrin secreting cells in which the beta-catenin/Tef-4-mediated transcriptional pathway is constitutively active or whether the colorectal cancer, adenomatous polyposis or metastasis displays progastrin-secreting cells and cells in which the beta-catenin/Tcf-4-mediated transcriptional pathway is constitutively active, comprises the step of detecting a mutation of the APC gene or of the beta-catenin gene or abnormal level of transcription of Tcf target genes.

Typically, the progastrin assay may be a time-resolved immunofluorimetric assay (TR-IFMA), as described for proGRP (Nordlund et al, 2007). This type of assay allows quantification in the range of 16 ng/l to 20,000 ng/l, values in the range of progastrin plasma levels measured in patients bearing a colorectal tumor. Two monoclonal antibodies may be used, one directed against the N-terminus of progastrin and the other against the C-terminus. The N-ter antibody may be biotinylated and used as the solid-phase antibody. The C-ter antibody may be labeled with Eu³⁺ and used as the tracer antibody.

Examples of inhibitors of progastrin-induced repression of ICAT may be selected within the group consisting of agent downregulating the expression of progastrin in colonic cells, antibody against progastrin, inhibitor of phosphatidylinositol 3 kinase (PI3K) and an inhibitor of Integrin-linked kinase (ILK).

For example, the inhibitor of progastrin-induced repression of ICAT is an agent downregulating the expression of progastrin in colonic cells. Typically, agent downregulating the expression of progastrin in colonic cells comprises a nucleic acid which interferes with the expression of progastrin.

Examples of such agents are antisense molecules or vectors comprising said antisense molecules. Antisense molecules are complementary strands of small segments of mRNA. Methods for designing effective antisense molecules being well known (see for example US 6,165,990), it falls within the ability of the skilled artisan to design antisense molecules able to downregulate the expression of progastrin in colonic cells. Further examples of agent downregulating the expression of progastrin are RNA interference (RNAi) molecules such as, for example, short interfering RNAs (siRNAs) and short hairpin RNAs (shRNAs). RNAi refers to the introduction of homologous double stranded RNA to specifically target a gene's product, in the present case progastrin, resulting in a null or hypomorphic phenotype. Methods for designing effective RNAi molecules being well known (see for review Hannon and Rossi Nature. 2004 Sep 16;431(7006):371-8), it falls within the ability of the skilled artisan to design RNAi molecules able to downregulate the expression of progastrin in colonic cells.

Examples of siRNAs able to downregulate the expression of progastrin in colonic cells are nucleic acid molecules which comprise one of the following sequences:

### siRNAs hPG:

Sense: 5'-GAAGAAGCCUAUGGAUGGATT-3' (SEQ ID N°27)
Antisense: 5'-UCCAUCCAUAGGCUUCUUCTT-3' (SEQ ID N°28)

### siRNAs mPG:

Sense: 5'-GAAGAGGCCUACGGAUGGTT-3' (SEQ ID N°29)
Antisense: 5'-CCAUCCGUAGGCCUCUUCTT-3' (SEQ ID N°30)

Examples of shRNAs able to downregulate the expression of progastrin in colonic cells are nucleic acid molecules which comprise one of the following sequences:

### shRNA hPG:

Sense: 5'-GAAGAAGCCTATGGATGGATTCAAGAGAAGGTAGGTATCCGAAGAAGTTTTTT-3' (SEQID N°1)
Antisense: 5'-AATTAAAAAACTTCTTCGGATACCTACCTTCTCTTGAATCCATCCATAGGCTTCTTCGGCC-3' (SEQ ID N°2)

One embodiment of the present disclosure relates to a medicament comprising a siRNA comprising one of the sequences selected from the group consisting of SEQ ID N°27, SEQ ID N°28, SEQ ID N°29 and SEQ ID N°30. Typically the medicament may comprise a pair of siRNAs. Examples of pairs of siRNAs are a first nucleic acid comprising SEQ ID N°27 and second nucleic acid comprising SEQ ID N°28 or a first nucleic acid comprising SEQ ID N°29 and second nucleic acid comprising SEQ ID N°30.

One embodiment of the present disclosure relates to a medicament comprising a shRNA comprising one of the sequences selected from the group consisting of SEQ ID N°1 and SEQ ID N°2. Typically the medicament may comprise a pair of shRNAs. Examples of pairs of shRNAs are a first nucleic acid comprising SEQ ID N°1 and second nucleic acid comprising SEQ ID N°2.

In an embodiment of the invention, the inhibitor of progastrin-induced repression of ICAT is an antibody against progastrin or a biologically active fragment or derivative thereof, which does not recognize amidated and glycine-extended forms of gastrin. The person skilled in the art will be aware of standard methods for production of such specific antibody. For example, specific antibodies or biologically active fragments or derivatives thereof may be generated by immunizing an animal with progastrin or progastrin fragments and by selecting the antibodies which bind to progastrin and which do not recognize amidated and glycine-extended forms of gastrin. In a preferred embodiment, the progastrin fragments are amino acid sequences which are specific to progastrin. The length of these sequences may be comprised between 8 and 15 amino acids. These sequences may comprise COOH-terminal amino acid residues or NH₂-terminal amino acid residues of progastrin which are not present in the gastrin forms G17 and G34-Gly. These sequences may comprise the cleavage site of prohormone convertase or of carboxypeptidase E.

In one embodiment of the present invention the antibody or biologically active fragment or derivative thereof, binds to the COOH-terminal 10-amino acid residues of progastrin or to the COOH-terminal 15-amino acid residues of progastrin. An example of COOH-terminal 10-amino acid residues of progastrin is FGRRSAEDEN (SEQ ID N°31). In an alternative embodiment, the antibody biologically active fragment or derivative thereof binds to the NH₂-terminal 40-amino acid residues of progastrin. Typically the antibody biologically active fragment or derivative thereof may bind to SWKPRSQQPDAPLGT (SEQ ID N°32). These amino acid sequences are specific to progastrin as they are not present in the gastrin forms G17, G17-Gly, G34 and G34-Gly.

The person skilled in the art will be aware of standard methods for production of both polyclonal and monoclonal antibodies and biologically active fragments and derivatives thereof. By biologically active fragment or derivative thereof, it is meant able to bind to the same epitope as the antibody, in the present case an epitope of progastrin. Antibody fragments, particularly Fab fragments and other fragments which retain epitope-binding capacity and specificity are also well known, as are chimeric antibodies, and "humanized" antibodies, in which structural (not determining specificity for antigen) regions of the antibody are replaced with analogous or similar regions from another species. Thus antibodies generated in mice can be "humanized" to reduce negative effects which may occur upon administration to human subjects. Chimeric antibodies are now accepted therapeutic modalities with several now on the market. The present invention therefore comprehends use of antibody specific for progastrin which include F(ab')₂, F(ab)₂, Fab, Fv and Fd antibody fragments, chimeric antibodies in which one or more regions have been replaced by homologous human or non-human portions. The person skilled in the art will also be aware that biologically-active antibody derivatives such as for example ScFv fragments and divalent ScFv-type molecules can be prepared using recombinant methods. The antibody may be labelled with a detectable marker, which is suitably a radioactive label, such as radioactive iodine, or may be a fluorescent or chemiluminescent label. A person skilled in the art will be able to select suitable radioactive, fluorescent or chemiluminescent labels.

One embodiment of the present invention relates to a medicament comprising an antibody or biologically active fragment or derivative thereof, which binds to the COOH-terminal 15-amino acid residues or to the NH₂-terminal 40-amino acid residues of progastrin.

Typically antibody or biologically active fragment or derivative thereof binds to FGRRSAEDEN (SEQ ID N°31) or to SWKPRSQQPDAPLGT (SEQ ID N°32).

In healthy humans, progastrin comprise less than 10% of circulating gastrins and no physiological role has been associated with progastrin. Consequently by specifically targeting progastrin with antibodies or biologically active fragments or derivatives thereof, side effects of the treatment are diminished if not avoided.

In a further embodiment of the present disclosure, the inhibitor of progastrin-induced repression of ICAT is an inhibitor of PI3K. Inhibitors of PI3K are well known. LY294002, wortmannin and quercetin are commonly used inhibitors of PI3K. US2006058321 and WO2004052373, for example disclose families of inhibitors of PI3K.

Antisense, RNAi molecules, dominant-negative forms of PI3K, antibodies against PI3K, biologically active fragments and derivatives thereof may also be used as inhibitors of PI3K.

In a further embodiment of the present disclosure, the inhibitor of progastrin-induced repression of ICAT is an inhibitor of ILK. Inhibitors of ILK are well known. KP-392 and QLT-0267 are commonly used inhibitors of ILK. For example, small molecule inhibitors of ILK are described in US6214813. Antisense inhibitors of ILK are described in US6177273. RNAi molecules, dominant-negative forms of ILK and antibodies against ILK, biologically active fragments and derivatives thereof may also be used as inhibitors of ILK.

Typically medicaments according to the disclosure comprise an inhibitor of progastrin-induced repression of the Inhibitor of beta-catenin and Tcf-4 binding (ICAT), together with a pharmaceutically-acceptable carrier. A person skilled in the art will be aware of suitable carriers. Suitable formulations for administration by any desired route may be prepared by standard methods, for example by reference to well-known text such as Remington; The Science and Practice of Pharmacy.

In another embodiment of the present disclosure, there is provided a method for screening for compounds for treating and/or preventing colorectal cancer, adenomatous polyposis or metastasis stemming from progastrin secreting cells, in which the beta-catenin/Tcf-4-mediated transcriptional pathway is constitutively active, comprising the following steps of:
a) providing progastrin secreting cells in which the beta-catenin/Tcf-4-mediated transcriptional pathway is constitutively active; and
b) adding the compound to be screened; and
c) selecting the compound which induces ICAT expression.

In an alternative embodiment of the present disclosure, there is provided a method for screening for compounds for treating and/or preventing colorectal cancer, adenomatous polyposis or metastasis stemming from progastrin secreting cells, in which the beta-catenin/Tcf-4-mediated transcriptional pathway is constitutively active, comprising the following steps of:
a) providing progastrin sensitive cells; and
b) adding progastrin to the medium containing the cells; and
c) adding the compound to be screened; and
d) selecting the compound which induces ICAT expression.

Typically the progastrin secreting cells or progastrin sensitive cells are colonic cells.

By progastrin sensitive cells, it is meant cells in which the beta-catenin/Tcf-4-mediated transcriptional pathway is active when progastrin is present in the culture medium.

The person skilled in the art will be aware of standard methods for implementing these methods. Typically ICAT expression may be determined by RT-quantitative PCR.

### FIGURE LEGENDS

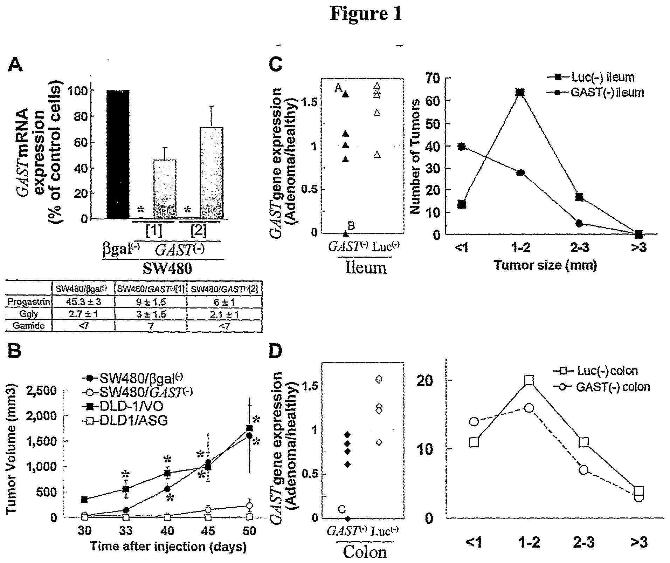

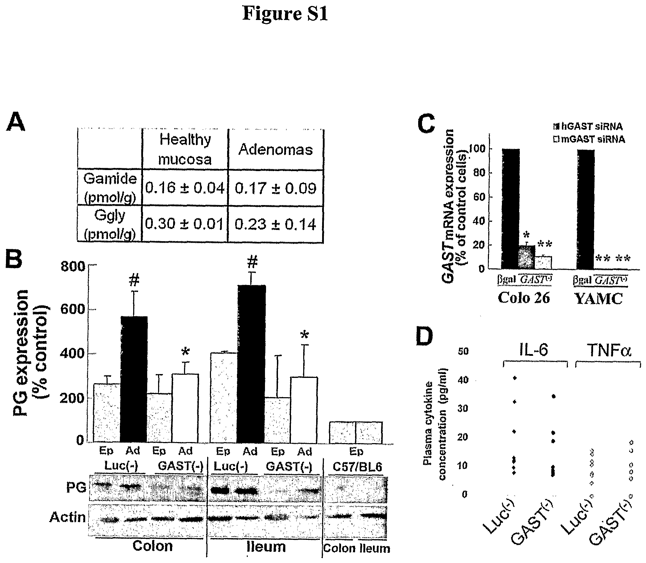

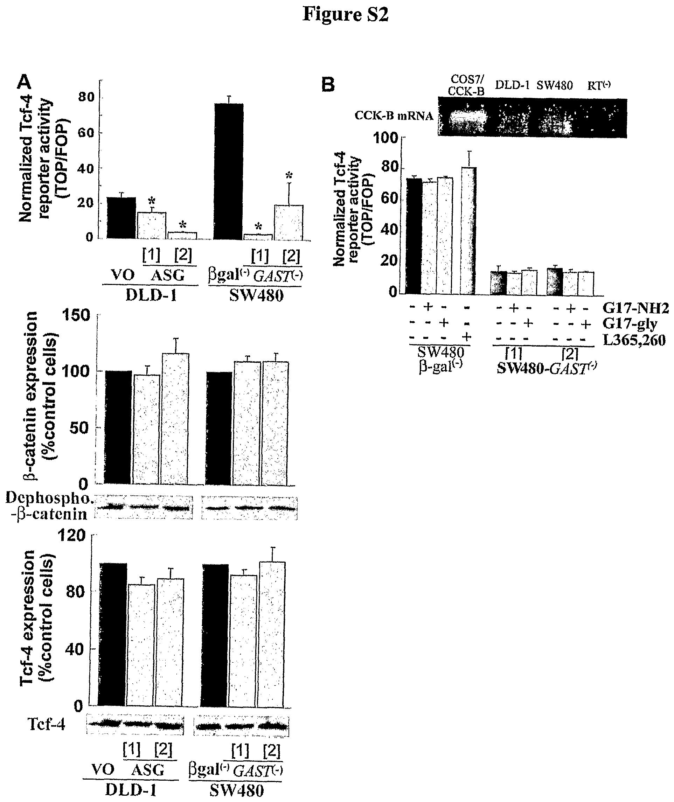

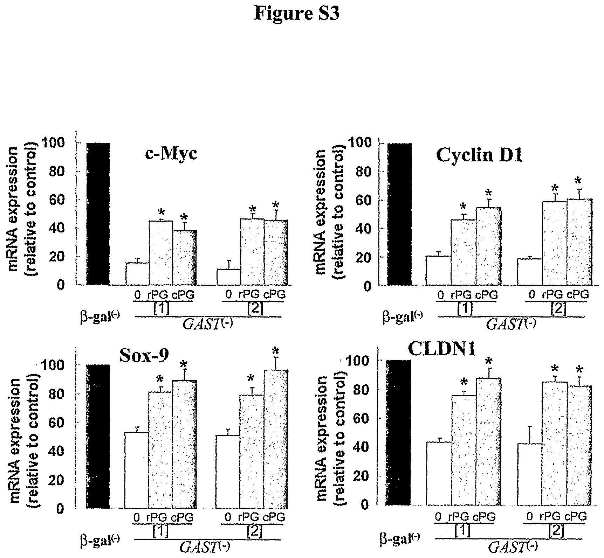

**Figure 1****. Progastrin depletion decreases tumorigenicity of human CRC cell lines in Balbc/ *nude* mice and inhibits spontaneous tumor development in the intestine of APCΔ14 mice. (A)** Histogram shows *GAST* mRNA quantification in SW480/βgal⁽⁻⁾ and SW480/GAST⁽⁻⁾ (clones [1] and [2], expressing or not a shRNA-insensitive preprogastrin cDNA (cPG)) cells. Results are expressed as a percentage of levels found in SW480/βgal⁽⁻⁾ (*, p<0.05 compared to SW480/GAST⁽⁻⁾, n=3). Bottom table, RIA quantification (pmol/l/24h) of progastrin, glycine-extended gastrin and amidated gastrin secreted by SW480/βgal⁽⁻⁾ and SW480/GAST⁽⁻⁾ cells. **(B)** Evolution of tumor volume over time after subcutaneous injection of DLD-1/VO, DLD-1/ASG, SW480/GAST⁽⁻⁾ or SW480/βgal⁽⁻⁾ cells in BALB/c *nude* mice. Means +/- s.e.m of 2 clones/cell line (4 mice/clone), (*, p<0.05 compared to DLD-1/VO or SW480/βgal⁽⁻⁾ cells, Student's t-test). **(C-D)** 3-month old APCΔ14 mice were treated for two weeks with siRNA targeting either the murine *Gast* or the Luciferase gene (9 mice/group). Results are expressed as a ratio between *Gast* gene expression in tumor/healthy mucosa in the ileum **(C)** or colon **(D)** (left panels), quantified using RT-QPCR. The number and size of adenomas were quantified after methylene blue staining in the ileum **(C)** and in the colon **(D)**, and are expressed as total number of tumors for each size group (right panels). *GAST* siRNA failed to bring down levels of *Gast* gene expression in sample A **(C)**, collected in a mouse with a high number of intestinal tumors. "B" and "C" correspond to mice that did not bear any colon or ileal tumor, respectively **(D)**.

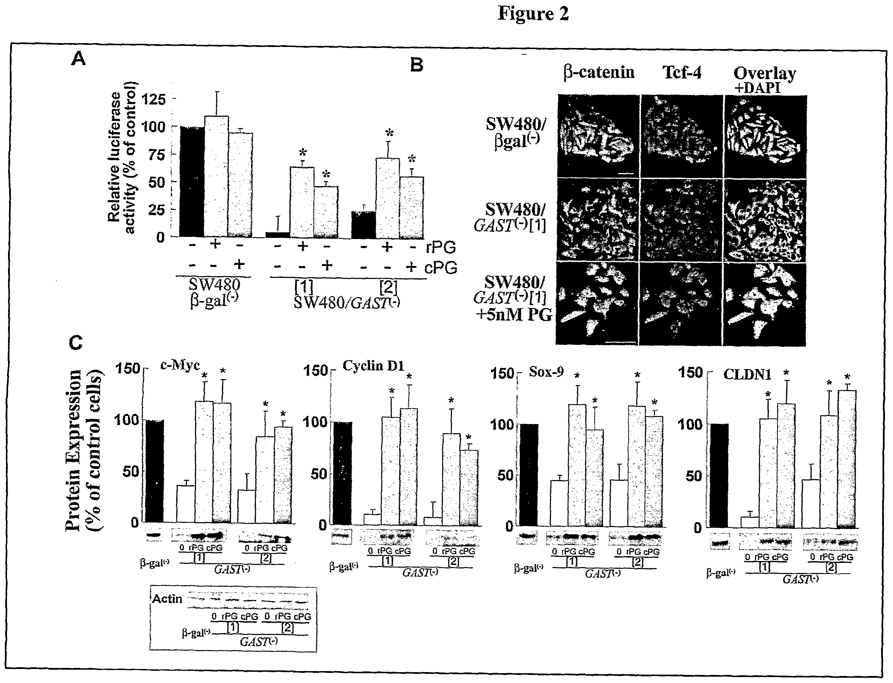

**Figure 2****. Progastrin stimulates B-catenin/Tcf-4 activity in human CRC cells (A)** Tcf-4 transcriptional activity was quantified using the TOP/FOP Luciferase reporter gene assay (Morin et al., 1997) in untreated SW480/βgal⁽⁻⁾ and SW480/GAST⁽⁻⁾ cells (clones [1] and [2]) (black bars), or in the same cells treated with 5nM recombinant progastrin for 72h (rPG, light grey bars) or transfected with a shRNA-insensitive preprogastrin cDNA (cPG, dark grey bars). Value are means ± s.e.m. of four similar experiments (*, p<0.05 compared to SW480/GAST⁽⁻⁾ cells). Levels of Tcf-4 and dephosphorylated β-catenin were similar in all of these cells, as shown in Figure S2. **(B)** Independent and overlaid confocal images of B-catenin (green), Tcf-4 (red), and DAPI (blue) in SW480/βgal⁽⁻⁾ cells and SW480/GAST⁽⁻⁾ cells with or without 5nM progastrin treatment for 72h. Bars represent 40µm. Higher magnification is used for progastrin-treated cells to provide better visualization of the partial reversion. (C) SW480/GAST⁽⁻⁾ clones [1] and [2] were treated or not as in **A.** Expression levels of actin and proteins encoded by the Tcf-4 target gene c-Myc, cyclin-D1, Sox-9 and CLDN1 were quantified by immunoblotting, using SW480/βgal⁽⁻⁾ cells as controls (see also mRNA expressions in supplemental Fig. S3). Values (standardized using actin as a loading control) are mean ± s.e.m. from three independent experiments. Student's t-test, p<0.05 (*) compared to SW480/GAST⁽⁻⁾ cells.

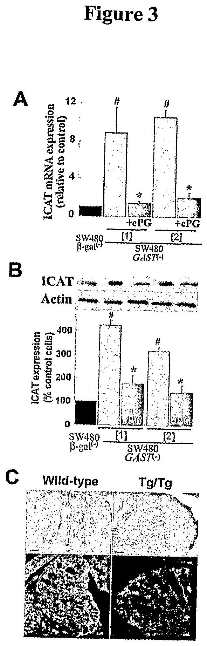

**Figure 3****. Progastrin down-regulates ICAT expression *in vitro* and *in vivo.* (A-B)** Progastrin-depleted SW480/GAST⁽⁻⁾ cells (clones [1] and [2]) were transfected or not with a codon-optimized, shRNA insensitive preprogastrin cDNA (cPG). ICAT mRNA levels were then quantified by RT-QPCR **(A)** in comparison with SW480/βgal⁽⁻⁾ cells, and expression of ICAT protein was analyzed by immunoblotting **(B)** in SW480/GAST⁽⁻⁾ before (light grey bars) or after transient transfection with cPG (grey bars), in comparison with SW480/β-gal⁽⁻⁾ (black bars). **(C)** ICAT expression was analyzed by immunohistochemistry and immunofluorescent staining in tissue sections obtained from the colonic epithelium of mice displaying intestinal-specific overexpression of progastrin (Tg/Tg) (Cobb et al., 2004), as well as on control mice from the same genetic background (Wild-type). Bars represent 40µm.

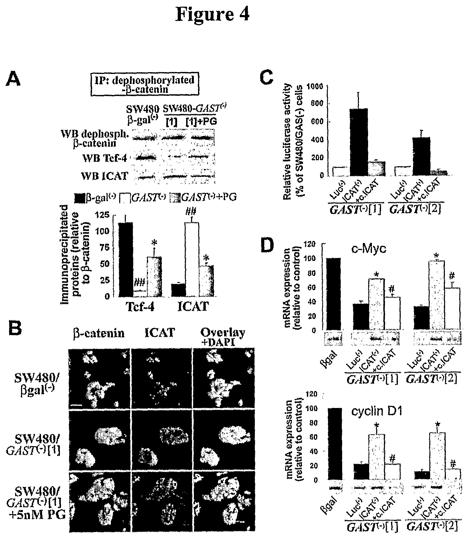

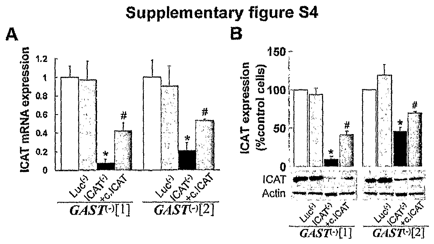

**Figure 4****. *De novo* ICAT expression is responsible for the decreased β-catenin-Tcf-**4 **activity in progastrin-depleted CRC cells. (A)** Top panel: dephosphorylated B-catenin immunoprecipitates from SW480/βgal⁽⁻⁾ and SW480/GAST⁽⁻⁾ +/- recombinant progastrin (rPG, 5nM, 72h) were probed for dephosphorylated β-catenin, Tcf-4 and ICAT. Bottom panel, quantification from 3 similar experiments performed on 2 independent clones (##p<0.01 and *p<0.05 compared to SW480/βgal⁽⁻⁾ and SW480/GAST⁽⁻⁾, respectively, Student's t-test). **(B)** Independent and overlaid confocal images of β-catenin, ICAT, and DAPI in SW480/βgal⁽⁻⁾ cells and SW480/GAST⁽⁻⁾ cells treated or not with 5nM progastrin for 72h. Bars represent 40µm. **(C-D)** Tcf-4 luciferase reporter assay (TOP/FOP) **(C)** and quantification of c-Myc (top) and cyclin D1 (bottom) mRNA and protein **(D)** were performed in SW480/GAST⁽⁻⁾/Luc⁽⁻⁾ and SW480/GAST⁽⁻⁾/ICAT⁽⁻⁾ cells with or without re-expression of a shRNA-insensitive ICAT cDNA (+cICAT).

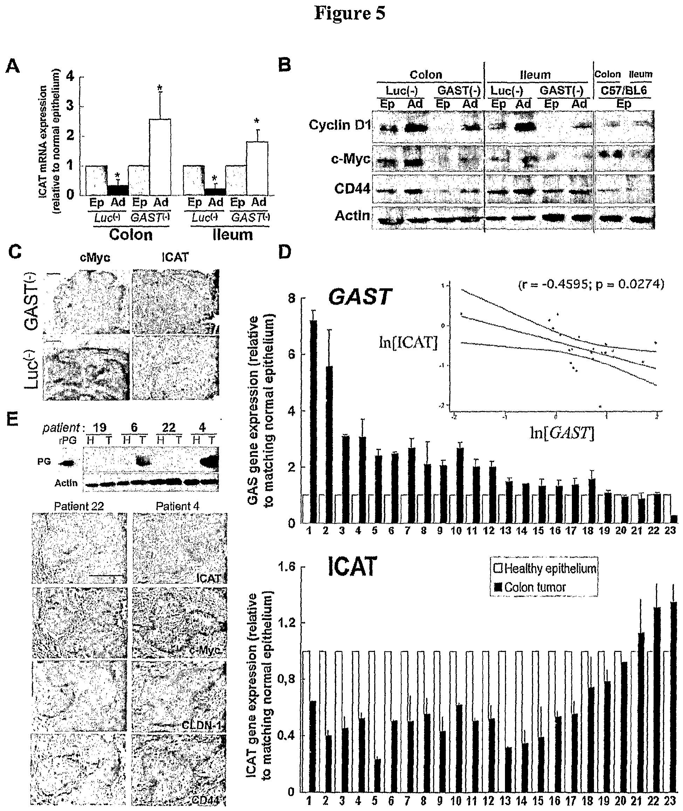

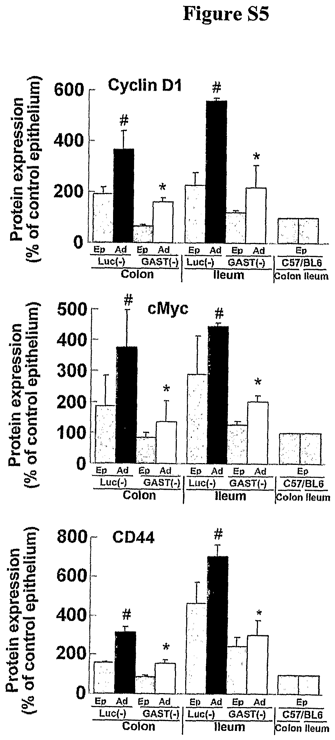

**Figure 5****. ICAT expression is inversely correlated to progastrin levels and** β-catenin-**Tcf-4 target gene expression in murine and human tumors. (A).** 3-month old APCΔ14 mice were treated for two weeks with siRNA targeting either the murine *GAST* gene or the Luciferase gene, as in Figure 1. Intestinal adenomas were processed for RNA extraction and ICAT mRNA expression was quantified (Ad) relative to the expression in matching epithelium (Ep) from the same animals (n=5). **(B)** Expression of the Tcf-4 targets cyclin D1, CD44 and C-Myc was analyzed using western immunoblotting in adenomas (Ad) and macroscopically intact epithelium (Ep) from the same animals, in comparison with intestinal epithelium of control mice from the same genetic background (see quantification on supplementary Figure S5). **(C)** Adenomas from APCΔ14 animals treated with *Luc* or *GAST*-specific siRNA were paraffin-embedded and processed for immunohistochemical detection of c-Myc and ICAT. Representative images are provided from *GAST*⁽⁻⁾ and *Luc*^{(*-*)}- adenomas collected in the ileum. Bars represent 20µm. (D) *GAST* and *ICAT* mRNA expressions in microdissected tumors (black bars) from 23 patients with CRC, expressed relative to the amounts found in their respective matching healthy epithelium (white bars), which were normalized to 1 for each patient. Values are means ± s.e.m of three independent experiments. Insert: log values of *GAST* and *ICAT* expression were plotted against each other, with the regression curve and its confidence interval. Spearman's correlation coefficient (r) is provided with its degree of significance (p). **(E)** Top panel: progastrin immunoblotting (IB) in healthy (H) or tumor (T) samples from 4 patients with CRC. Bottom panel: representative immunohistochemistry (IHC) for ICAT, c-Myc, claudin-1, and CD44 patients with either high (patient 4) or low (patient 22) tumor progastrin expression (positive control in WB was recombinant progastrin (rPG); exposure time was 20 sec for positive control, and 10 min for human samples). Similar IB and IHC results were obtained on samples from the 11 patients tested. Bar represents 20µm.

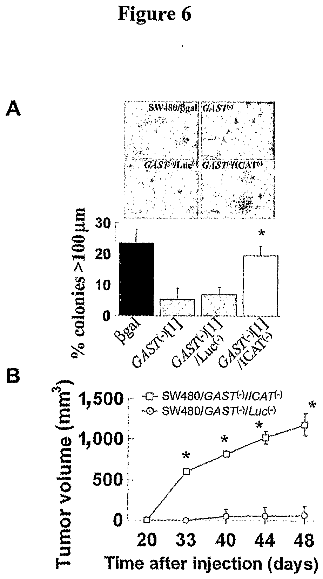

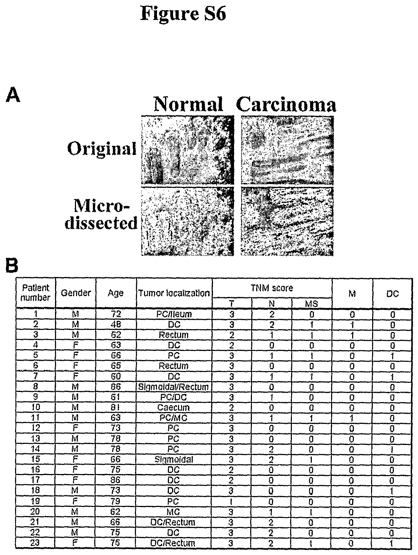

**Figure 6****. Repression of ICAT is essential to the tumor-promoting role of progastrin in human CRC cells. (A)** Top panel: Down-regulation of ICAT reverses the decreased growth-rate of progastrin-depleted cells in soft agar, as shown by the marked differences in colony size between SW480/βgal⁽⁻⁾,SW480/GAST⁽⁻⁾, SW480/GAST⁽⁻⁾/ICAT⁽⁻⁾ and SW480/GAST⁽⁻⁾/Luc⁽⁻⁾ cells. Bottom panel: quantification of 3 similar experiments, expressed as a mean ± s.e.m. from ten randomly chosen fields per clone. **(B)** SW480/GAST⁽⁻⁾/Luc⁽⁻⁾ and SW480/GAST⁽⁻⁾/ICAT⁽⁻⁾ cells were injected in the posterior leg of BALB/c *nude* mice, and tumor growth was measured regularly. Mean ± s.e.m. of 4 clones each. *, p<0.05 compared to SW480/GAST⁽⁻⁾/Luc⁽⁻⁾ cells, Student's t-test.

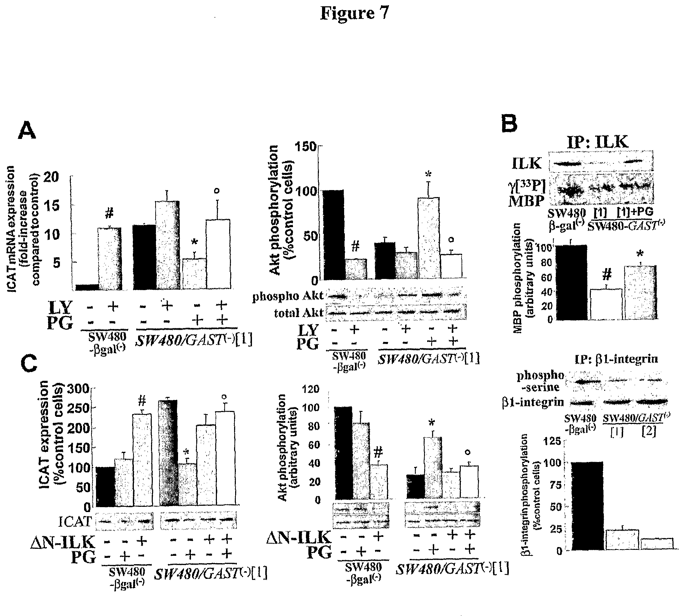

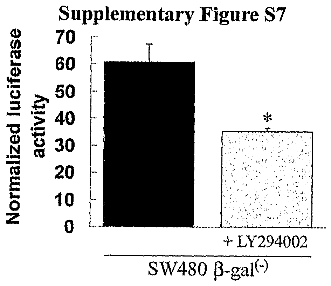

**Figure 7** **Activation of the PI3K/ILK pathway is essential to the repression of ICAT by progastrin (A)** PI3K activation is essential for progastrin-induced ICAT repression in human CRC cells. ICAT mRNA expression was measured as described before (Left), and phosphorylation of Akt/PKB was analyzed by Western blotting (Right) in SW480/βgal⁽⁻⁾ and SW480/GAST⁽⁻⁾ cells with or without treatment with 5nM progastrin (PG) and/or the PI3Kinase inhibitor LY-294002 (LY), as indicated. **(B)** ILK expression and activity are reduced in progastrin-depleted human CRC cells. Top panel, ILK expression (Western blot) and enzymatic activity *(in vitro* phosphorylation of Myelin Basic Protein (MBP)) were analyzed after ILK immunoprecipitation from SW480/βgal⁽⁻⁾ and SW480/GAST⁽⁻⁾ cell lysates, with or without treatment with recombinant progastrin (5nM, 72h). Middle panel shows the quantification of ILK activity, performed on three independent experiments after correcting for variations of ILK expression in cells described above. In the bottom panel, phosphorylation of the ILK target β1-Integrin was analyzed by sequentially probing β1-Integrin-immunoprecipitates with an anti-phosphoserine antibody and an antibody against β1-Integrin. ILK, β1-Integrin, and MBP phosphorylation were never detected when lysates were immunoprecipitated with preimmune rabbit serum (not shown). (C) ILK activation is essential for progastrin-mediated ICAT repression in human CRC cells. Expression of ICAT mRNA (Left panel) and phosphorylation of Akt/PKB on Ser473 (Right panel) were quantified in SW480/βgal⁽⁻⁾ and SW480/GAST⁽⁻⁾ cells transfected or not with a dominant negative ILK (ΔN-ILK), and with or without treatment with 5nM exogenous progastrin (PG) as indicated. For all panels, p<0.05 compared to SW480/βgal⁽⁻⁾ (#),SW480/GAST⁽⁻⁾ (*), or progastrin-treated SW480/GAST⁽⁻⁾ (°) cells.

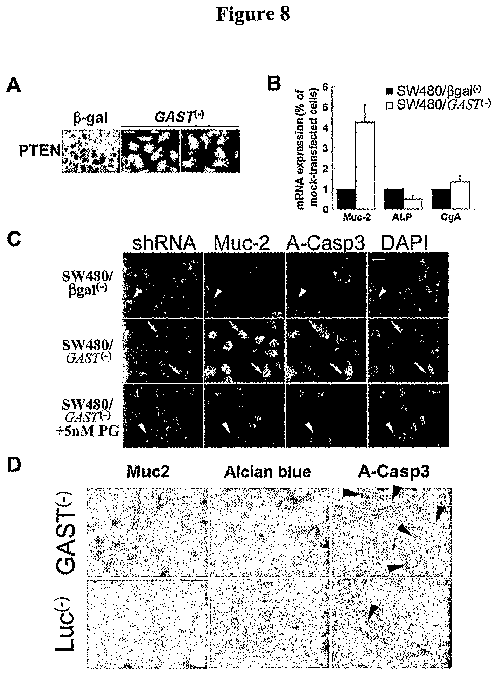

**Figure 8****. Progastrin depletion induces differentiation and apoptosis of human CRC cells *in vitro* and promotes tumor differentiation in the intestine of APCΔ14 mice *in vivo.* (A)** Representative experiment showing the nuclear localization of PTEN in two clones of SW480/GAST⁽⁻⁾ but not in SW480/(βgal⁽⁻⁾ cells. **(B)** Quantification of Muc-2, intestinal alkaline phosphatase (ALP), and chromogranin A (CgA) mRNA expression in SW480 cells 48h after transfection with siRNAs targeting *GAST* or β-galactosidase. **(C)** Muc-2 expression and caspase-3 activation in SW480 CRC cells after transient transfection with GAST-specific but not β-galactosidase-specific siRNAs. Cells were transfected with rhodamine-tagged siRNAs (red) targeting *GAST* (arrows) or β-galactosidase (arrowheads), and treated or not with 5nM recombinant progastrin. Muc-2 expression (A488, green) and activated caspase-3 (Cy-5, shown here in red) were detected by immunofluorescent staining, and cell nuclei were stained with DAPI (blue). Similar results were obtained with DLD-1 cells. Bars represent 20µm. **(D).** 3-month old APCΔ14 mice were treated for two weeks with siRNA targeting either the murine *GAST* gene or the Luciferase gene, as in Figure 1. Intestinal adenomas were paraffin-embedded and processed for immunohistochemical detection of mucus-producing cells (Muc2 and Alcian blue) and cells engaged in the apoptotic pathway (activated caspase 3, black arrowheads). Representative images are provided from *GAST⁽⁻⁾* and *Luc⁽⁻⁾* - adenomas collected in the ileum. Bars represent 20µm.

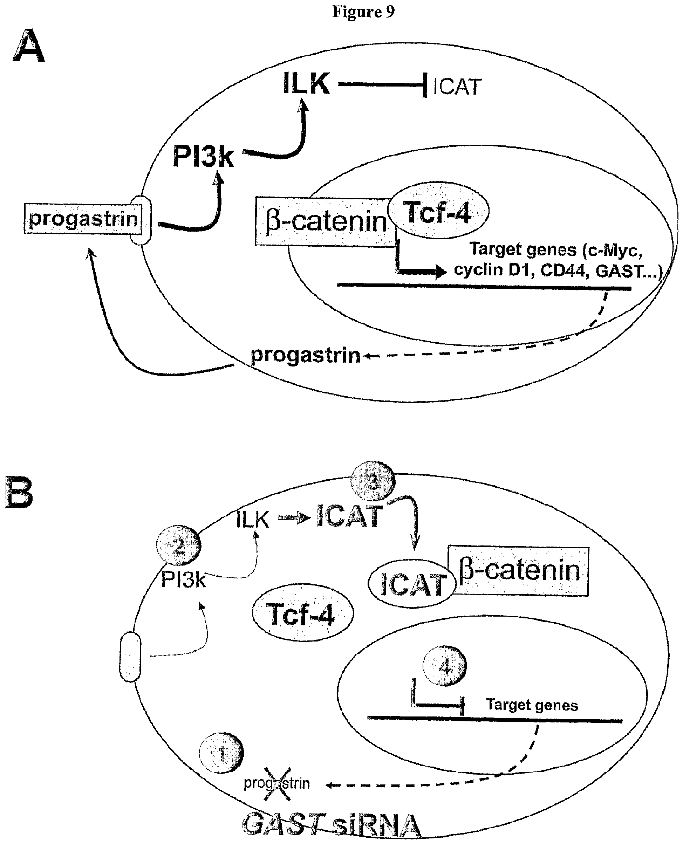

**Figure 9****. Signaling pathway connecting progastrin depletion with a decreased activation of the β-catenin/Tcf-4 transcriptional complex in APC-mutated colorectal carcinoma cells. (A).** CRC cells produce and secrete endogenous progastrin, which act in turn on tumor cells to activate PI3 kinase and ILK, thereby inducing a repression of ICAT and facilitating the amplification of β-catenin/Tcf-4 transcriptional activity initiated by the APC mutation. This process results in maximal transcription of target genes such as the gene encoding progastrin, thus creating a self-amplifying activation loop. **(B)** Inhibition of progastrin action, such as that induced by siRNA silencing of the GAST gene (①), markedly reduces the activity of PI3 kinase and ILK (②), resulting in a strong up-regulation of ICAT (③). Competition of this small inhibitor with Tcf-4 for binding to β-catenin is sufficient to strongly down-regulate transcription of Tcf-4 targets in tumor cells (④).

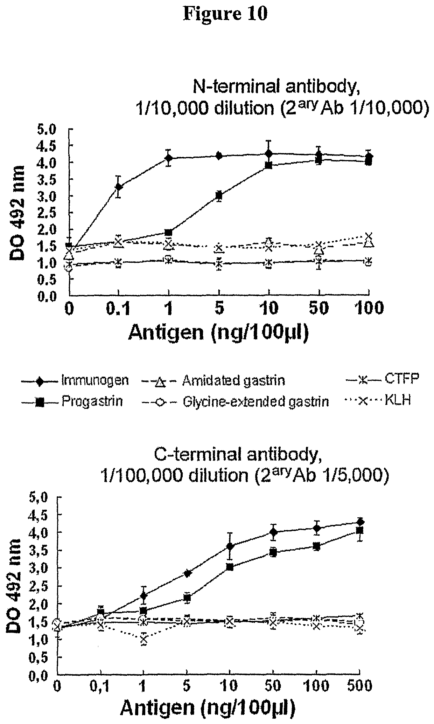

**Figure 10****.** Increasing concentrations of antigen was coated onto 96-well plates as indicated (for details, cf. "Methods" section), followed by incubation with selective antibodies against the N-terminal (top panel) or C-terminal (bottom panel) extremities of progastrin(1-80). Antigens tested were the original immunogens used to generate the antibodies (Top panel: SWKPRSQQPDAPLGT, bottom panel: FGRRSAEDEN), amidated gastrin17, glycine-extended gastrin17, the C-terminal flanking peptide of progastrin (SAEDEN), and Keyhole Limpet Hemocyanin (KLH), which was used as a carrier protein during the immunization process. Results are expressed as direct 492nm readings after incubation with secondary antibodies and OPD substrate.

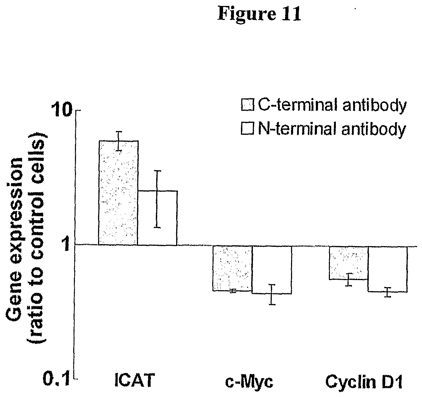

**Figure 11****.** SW480 colorectal carcinoma cells were treated for 30 hours with a control antibody or antibodies directed against the C-terminal or N-terminal extremities of progastrin (1/5,000 dilution), as described in the Methods section. After cell lysis, expression of the mRNA for ICAT, c-Myc and cyclin D1 was quantified using RT-qPCR. Results are expressed as ratio between the expression in cells treated with C-terminal (grey bars) or N-terminal (white bars) progastrin antibodies and cells treated with the control antibody, in which each gene expression level was fixed at a value of 1 (represented here by the horizontal axis).
**Figure S1. Overexpression of progastrin in the adenomas of APCΔ14 mice and lack of proinflammatory response after a two-week treatment with siRNA selective for the murine GAST gene. (A)** Amidated and glycine-extended gastrin levels in the healthy intestinal mucosa and the adenomas of 3.5-months old APCΔ14 mice, quantified by radioimmunoassay. **(B)** Representative progastrin immunoblot in healthy intestinal mucosa and adenoma protein extracts (50µg) from 3.5-month old APCΔ14 mice treated with *Luc* or *GAST* gene-specific siRNA, in comparison with control mice from the same genetic background (No RIA is currently available to quantify murine progastrin). Films were exposed for 30 min. Histogram, quantification of PG in adenomas collected in the colon and ileum of 4 animals per group, expressed relative to colon and ileum of C57/BL6 animals. (for each intestinal segment, p<0.05 compared to respective values in C57/BL6^{#} or in APCΔ14 *Lue⁽⁻⁾*)* **(C)** Colo-26 murine CRC cells and Young adult mouse colon (YAMC) cells were transfected with siRNA directed against the murine *(mGAST* siRNA) or the human *(hGAST* siRNA) *GAST* sequence, and expression of *GASTmRNA* was quantified using RT-QPCR. **(D)** Scatter plot summarizing the levels of plasma IL-6 (black) and TNFα (white) in mice treated with *Luc* or *GAST* siRNA. No significant differences were noticed between these two groups, where levels of both cytokines reflect the absence of inflammation.
**Figure S2. Progastrin, but not amidated or glycine-extended gastrin, stimulates β-catenin/Tcf-4 transcriptional activity in CRC cells. (A)** (Top panel) Tcf-4 transcriptional activity was quantified using the TOP/FOP Luciferase reporter gene assay (31) in DLD-1/VO, DLD-1/ASG (clones [1] and [2]), SW480/βgal⁽⁻⁾ and SW480/GAST⁽⁻⁾ (clones [1] and [2]) cells. Value are means ± s.e.m. of 4 similar experiments. Levels of dephosphorylated (B-catenin (Middle panel) and Tcf-4 (Lower panel) were similar in all of these cell lines, as shown by immunoblotting (using actin as a loading control). **(B)** Human amidated gastrin (CCK-B) receptor mRNA was detected by RT-PCR in samples from COS-7 cells transfected with the CCK-B receptor construct, but not in DLD-1 and SW480 cells or in negative control (COS-7/CCK-B without reverse transcription).
**Figure S3. mRNA expression of the Tcf-4 target genes c-Myc, cyclin D1, Sox-9 and CLDN1 is regulated by progastrin in human CRC cells.** SW480/*GAST*⁽⁻⁾ clones [1] and [2] were treated or not with 5 nM progastrin (rPG) for 72h or transfected with a codon-optimized, shRNA insensitive preprogastrin cDNA (cPG). mRNA expression of the Tcf-4 targets c-Myc, cyclin D1, Sox9 and claudin-1 (a list of Tcf-4 target genes is available in (9)) were quantified by RT-QPCR, using SW480/βgal⁽⁻⁾ cells as controls. Values are mean ± s.e.m. from three independent experiments. Student's t-test, p<0.05 (*) compared to SW480/*GAST*⁽⁻⁾ cells.
**Figure S4. Experimental modulation of ICAT expression in CRC cells.** ICAT mRNA **(A)** and protein levels **(B)** were quantified in two clones each of progastrin-depleted CRC cells (SW480/*GAST*⁽⁻⁾, white bars), transfected or not with control shRNA (SW480/*GAST*⁽⁻⁾/Luc⁽⁻⁾, grey bars) or shRNA selective for ICAT (SW480/*GAST*⁽⁻⁾/ICAT⁽⁻⁾ cells, black bars), as well as in SW480/*GAST*⁽⁻⁾/ICAT⁽⁻⁾ cells transiently transfected with a codon-optimized, shRNA insensitive ICAT cDNA (+c.ICAT) (dark-grey bars). (p<0.05 compared to SW480/*GAST*⁽⁻⁾/Luc⁽⁻⁾ (*), or compared to SW480/*GAST*⁽⁻⁾/ICAT^{(-)(#)}, student's t-test, n=3)
**Figure S5. Elevated expression of Tcf-4 target genes in intestinal adenomas from APCΔ14 mice is reduced after progastrin-targeting siRNA treatment.** 3-month old APCΔ14 mice were treated for two weeks with siRNA targeting either the murine *GAST* gene or the Luciferase gene, as in Figure 1. Intestinal adenomas were processed for protein extraction, and expression of the Tcf-4 targets cyclin D1 (Top), c-Myc (Middle) and CD44 (Bottom) was quantified after Western immunoblotting in adenomas (Ad) and macroscopically intact epithelium (Ep) from the same animals, in comparison with intestinal epithelium of control mice from the same genetic background (Representative immunoblot is shown on Figure 5B). Values represent means ± sem of adenomas from 4 animals/group. For each intestinal segment, p<0.05 compared to respective values in C57/BL6^{#} or in APCΔ14 *Luc⁽⁻⁾***)*.
**Figure S6. Patient population used for Laser capture microdissection of colon samples. (A)** Representative photomicrographs of typical healthy and tumoral colon tissues prior to (Original) and after Laser Capture Microdissection (Microdissected). **(B)** Characteristics of the patients population used to determine *GAST* and *ICAT* gene expression levels, including the pathology scoring of their tumor according to the TNM classification. Tumor localization is represented as PC (proximal colon), MC (Middle colon), DC (distal colon), Sigmoidal, or Rectum. Metastasis at the time of surgery (MS), metastasis at a later stage (M), and Deceased patients (DC) are provided, with the following code (0: No; 1: Yes)
**Figure S7. Pharmacological inhibition of PI3K inhibits Tcf-4 transcriptional activity in SW480/βgal⁽⁻⁾ cells.** SW480/βGal⁽⁻⁾ cells were treated or not with 10 µM LY294002 and Tcf-4 activity was quantified as described above. Values are mean ± s.e.m. from three independent experiments. Student's t-test, p<0.05 (*) compared to untreated SW480/βgal⁽⁻⁾ cells.

### EXAMPLES

### Example 1

In the following description, all molecular biology experiments for which no detailed protocol is given are performed according to standard protocol.

### SUMMARY

Background and aims: Aberrant activation of the β-catenin/Tcf-4 transcriptional complex represents an initiating event for colorectal carcinogenesis, shifting the balance from differentiation towards proliferation in colonic crypts. Here, we assessed whether endogenous progastrin, encoded by a target gene of this complex, was in turn able to regulate β-catenin/Tcf-4 activity in APC-mutated cells, and we analyzed the impact of topical progastrin depletion on intestinal tumor growth in vivo.

Methods: Stable or transient RNA silencing of the GAST gene was induced in human tumor cells and in mice carrying a heterozygous Apc mutation (APCΔ14), which overexpress progastrin but not amidated or glycine-extended gastrin.

Results: Depletion of endogenous progastrin production strongly decreased intestinal tumor growth in vivo, through a marked inhibition of constitutive, β-catenin/Tcf-4 activity in tumor cells. This effect was mediated by the de novo expression of the Inhibitor of β-catenin and Tcf-4 (ICAT), resulting from a down-regulation of Integrin-linked kinase (ILK) in progastrin-depleted cells. Accordingly, ICAT down-regulation was correlated with progastrin overexpression and Tcf-4 target gene activation in human colorectal tumors, and ICAT repression was detected in the colon epithelium of tumor-prone, progastrin-overexpressing mice. In APCΔ14 mice, siRNA-mediated progastrin depletion not only reduced intestinal tumor size and numbers, but also increased goblet cell lineage differentiation and cell apoptosis in the remaining adenomas.

Conclusions: Thus, depletion of endogenous progastrin inhibits the tumorigenicity of APC-mutated CRC cells in vivo by promoting ICAT expression, thereby counteracting Tcf-4 activity. Progastrin targeting strategies should provide an exciting prospect for the differentiation therapy of colorectal cancer.

### INTRODUCTION

Various strategies aiming to reduce tumor development through the induction of tumor cell differentiation have been developed in recent years, providing very promising results in animal models and in human patients. In particular, the use of all-trans-retinoic acid has proven extremely useful in the treatment of acute promyelocytic leukemia (Lallemand-Breitenbach et al., 2005; Wang and Chen, 2000), and is considered as a promising approach to force tumor differentiation in advanced thyroid cancer (Coelho et al., 2005). In the intestine, great hopes have been placed on agents modulating two major pathways involved in the control of cell proliferation and differentiation, namely the Notch cascade and the Wnt/B-catenin/Tcf-4 pathway (Radtke and Clevers, 2005; van Es and Clevers, 2005).

However, since a fine-tuned interplay between Notch and Wnt activation is essential for the homeostasis of the intestinal crypts, approaches aimed at directly targeting these signaling pathways in colon tumors are likely to have adverse effects on overall intestinal physiology. Ideally, such strategies would greatly benefit from the capacity to identify targets that are selectively activated, or strongly stimulated in tumor cells, while being absent or inactivated in the surrounding tissues. Among the targets specifically activated in intestinal tumor cells, partially processed Gastrin-gene *(GAST)* products, such as glycine-extended gastrin and progastrin, offer an interesting prospect for the selective targeting of tumor growth. Indeed, the *GAST* gene itself is a target of both Tcf-4 and K-Ras (Chakladar et al., 2005; Koh et al., 2000), two pathways that are frequently activated and act synergistically in colorectal cancers (Janssen et al., 2006), and these GAST-derived peptides seem to stimulate proliferation via an autocrine/paracrine loop on tumor cells (Hollande et al., 1997; Hollande et al., 2003). The proliferative effect of Ggly was discovered during the last few years (Seva et al., 1994) and more recently, progastrin was shown to stimulate proliferation (Ottewell et al., 2005; Singh et al., 2000) and to modulate epithelial cell/cell adhesion and migration (Hollande et al., 2003; Hollande et al., 2005). In addition, conflicting results emerged from experiments using animals with a targeted deletion of the *GAST* gene. Indeed, this deletion strongly enhanced tumorigenesis induced by a chemical carcinogen, azoxymethane (Cobb et al., 2002), whereas it resulted in a decreased number of polyps in the genetic background of APc^{+/min} mice (Koh et al., 2000). Yet, they provided little information concerning the potential for treatments impairing the function of these peptides to slow-down or revert the growth of preexisting tumors. Critically, progastrin is produced and secreted in significant quantities by almost 80% of human colorectal tumors and tumor cell lines, while its secretion falls mostly below detection levels under physiological conditions (Ciccotosto et al., 1995; Konturek et al., 2002; Siddheshwar et al., 2001; Van Solinge et al., 1993). Therefore, since the constitutive activation of the Wnt signaling pathway represents a hallmark of sporadic CRC, as well as of the hereditary condition of adenomatous polyposis (Fodde et al., 2001), and since the *GAST* gene is itself a target of Tcf-4, we investigated whether progastrin was able, in turn, to modulate APC mutation-driven tumorigenesis, and whether targeting progastrin could represent a relevant strategy to reduce the growth of intestinal tumors.

We show that RNA interference-mediated progastrin depletion was capable of inhibiting tumor growth induced by a mutation of the *APC* gene *in vivo.* We then demonstrate that this inhibition reflected the capacity of progastrin to modulate the level of β-catenin /Tcf-4 transcriptional activity in tumor cells, in spite of its constitutive activation triggered by the *APC* mutation. *De novo* expression of the inhibitor of β-catenin and Tcf-4 binding (ICAT) was instrumental to inhibit the β-catenin /Tcf-4 transcriptional pathway and to decrease tumorigenicity in progastrin-depleted tumor cells, while repression of ICAT was detected in progastrin-overexpressing mouse colonic mucosa, and in human and mouse intestinal tumors. Finally, treatment with GAST-specific siRNA induced a terminal differentiation towards the goblet cell lineage, not only of human CRC cells *in vitro,* but also of the progastrin-secreting intestinal adenomas of mice carrying a heterozygous mutation of the *Apc* gene.

### RESULTS

### Progastrin depletion reverses tumor growth induced by APC mutations in vivo

To assess whether a strategy aimed at selectively targeting endogenous progastrin in preexisting tumors would be able to antagonize Tcf-4-promoted tumor growth *in vivo,* we used Balbc/*nude* mice xenografts from the colorectal cell line SW480 (Morin et al., 1997), as well as a mouse model of spontaneous intestinal tumorigenesis, APCΔ14 (Colnot et al., 2004). Similar to what is found in a majority of human colorectal tumors (Korinek et al., 1997), these two experimental models bear mutations of APC and consequently display a constitutive activation of the β-catenin/Tcf-4 pathway.

Control SW480/βgal⁽⁻⁾ and DLD-1/VO cells were shown to express high progastrin levels but little of the amidated and glycine-extended forms of gastrin (Table 1). Within 7 weeks of subcutaneous injection in Balbc/*nude* mice, these cells produced large tumors (Figure 1B). In contrast, the ability to form tumors within the same timespan was greatly reduced when progastrin secretion was down-regulated by stable expression of short hairpin RNA (shRNA), directed against the *GAST* gene (SW480/ *GAST*⁽⁻⁾ cells, Figure 1A-B). Similar results were obtained after injection of previously characterized (Hollande et al., 2003) DLD-1 colorectal cancer cells expressing antisense *GAST* gene cDNA (Figure 1 B).

**Table 1:**

| | DLD-1/VO | DLD-1/ASG[1] | DLD-1/ASG[2] |
|---|---|---|---|
| Progastrin | (396±11) | (19±3) | (23±1.5) |
| Ggly | (310±7) | (18.6±2) | (17±2) |
| Gamide | (<7) | (<7) | (<7) |
| | SW480/βgal⁽⁻⁾ | SW480/GAS⁽⁻⁾ [1] | SW480/GAS⁽⁻⁾ [2] |
| Progastrin | 45.3±3 | 9±1.5 | 6±1 |
| Ggly | 2.7±1 | 3±1.5 | 2.1±1 |
| Gamide | <7 | 7 | <7 |

Next, we demonstrated that progastrin inhibition decreased the growth of preexisting intestinal tumors in a more pathologically relevant context, using APCΔ14 mice. These animals have a similar truncating mutation of the *Apc* gene to that found in human familial adenomatous polyposis and sporadic colorectal cancer patients. They also partially recapitulate the intestinal epithelium phenotype found in these patients, since they develop spontaneous tumors in the ileum but also more colon tumors than the APCmin^{+/-} model (Colnot et al., 2004). Three-month-old *APCΔ*14 mice always display multiple adenomas in the ileum and colon (data not shown), and progastrin levels, but not those of amidated or glycine-extended gastrin, were found to be elevated in these adenomas (Figure S1). These animals therefore provided us with an ideal *in vivo* model to specifically study the role of progastrin modulation on Tcf-4-promoted tumor growth.

Daily treatment for two weeks with siRNA directed against the murine *GAST* gene (*APCΔ*14/*GAST*⁽⁻⁾*)*, previously shown to be effective in mouse cells (Figure S1), induced a reduction in *GAST* gene and progastrin expression in intestinal adenomas, compared to that found after treatment with a Luciferase-specific siRNA (APCΔ14/ *Luc*⁽⁻⁾) (Figure 1C-D, and S1). In correlation with the reduced *GAST* expression, a significant reduction in tumor size was detected in the intestine of APCΔ14/ *GAST*⁽⁻⁾ animals, compared to controls (Chi-square, p<0.0001, n=9 per group). This reduction was particularly marked in the ileum (Figure 1C), but a similar trend was visible in the colon, although not reaching significance due to the smaller number of tumors located in this area (Fischer's exact test, p=0.228) (Figure 1D). In addition, a 20% decrease in the total number of intestinal tumors was detected overall in APCΔ14/ *GAST*⁽⁻⁾ mice compared to control animals. In particular, this reduction was drastic in two animals of the APCΔ14/ *GAST*⁽⁻⁾ group, where the ileum or colon were found to be tumor-free. In contrast, the GAST-specific siRNA treatment failed to down-regulate *GAST* gene levels in one mouse from the same group, and this animal displayed similar number and size of tumors to those found in controls (Figure 1C). Therefore, although the efficiency of the siRNA-mediated down-regulation of progastrin production varied among APCΔ14/ *GAST*⁽⁻⁾ animals, tumor sizes and numbers were consistently decreased in mice displaying reduced progastrin levels.

Taken together, these results show that progastrin secretion is essential for the tumorigenicity of APC-mutated human CRC cells, and that a treatment inhibiting progastrin production *in vivo* strongly decreased tumor growth in a mouse model recapitulating the early stages of adenomatous polyposis and human colorectal tumorigenesis.

### Progastrin modulates the level of β-catenin/Tcf-4 transcriptional activity in APC-mutated intestinal tumor cells.

Since we showed that targeting progastrin inhibited tumor growth in two different *APC*-mutated intestinal tumor models, we postulated that this effect was due to the capacity of progastrin to modulate the activity levels of the β-catenin/Tcf-4 transcriptional complex, known to be constitutively activated by the APC mutation. This activity, quantified by transcription of a luciferase reporter gene (TOP/FOP) (Korinek et al., 1997), was indeed elevated in control cells (SW480/βgal⁽⁻⁾), but was significantly inhibited in progastrin-depleted clones (Figure 2A), without detectable modulation of β-catenin or Tcf-4 levels (Figure S2). A similar result was obtained in previously established (Hollande et al., 2003) DLD-1 cells expressing an antisense *GAST* cDNA (Figure S2). Treatment with 5nM recombinant progastrin, but also endogenous progastrin produced by expression of a codon-optimized, shRNA insensitive, preprogastrin construct in SW480/*GAST*⁽⁻⁾ clones, were found to restore a strong level of transcription of the luciferase reporter gene, confirming that progastrin is able to stimulate this transcriptional pathway (Figure 2A). In addition, immunofluorescent staining indicated that the reduction of β-catenin/Tcf-4 activity correlated with a marked reduction in the nuclear amounts of Tcf-4 and dephosphorylated β-catenin in SW480/ *GAST*⁽⁻⁾ cells, and treatment of these cells with 5 nM recombinant progastrin partially restored the nuclear compartmentation of β-catenin and Tcf-4 (Figure 2B). Similarly, the expression of several Tcf-4 target genes (c-myc, cyclin D1, Sox-9 and claudin-1) (Blache et al., 2004; van de Wetering et al., 2002) was strongly down-regulated after progastrin-depletion, and was significantly stimulated by reexpression of progastrin or treatment with the recombinant peptide (Figures 2C and S3). In contrast, treatment with amidated or glycine-extended gastrin17, or with the amidated gastrin (CCK-B) receptor antagonist L365, 260, did not affect β-catenin/Tcf-4 activity in these cells (Figure S2), indicating that short processed forms of gastrin are unable to mimick the regulation of β-catenin/Tcf-4 activity by progastrin in these cells.

The above data clearly demonstrates that progastrin produced by SW480 CRC cells stimulates the activity of the β-catenin/Tcf-4 complex. It also shows that blocking progastrin production results in a strong inhibition of this transcriptional pathway, in spite of its constitutive activation in *APC*-mutated cells.

### The inhibitor of β-catenin and Tcf-4 (ICAT) is the molecular switch mediating the modulation of β-catenin/Tcf-4 activity by progastrin.

In view of the mutated status of the APC gene in the CRC cells used, and since the nuclear compartmentation of Tcf-4 and β-catenin was significantly altered in progastrin-depleted cells (Figure 2A), we hypothesized that the decreased β-catenin/Tcf-4 activity could result from an increased interaction of β-catenin with another of its partners. One of these partners, ICAT (Inhibitor of β-catenin and Tcf-4), was recently identified as a direct inhibitor of the association between these two proteins (Tago et al., 2000), and is responsible for reduced proliferation and increased cell death when overexpressed in DLD-1 and SW480 cells (Sekiya et al., 2002). Progastrin depletion induced a strong expression of ICAT mRNA and protein in SW480/ *GAST*⁽⁻⁾ cells (Figure 3A and B), while the expression of another binding partner of β-catenin, E-cadherin, was unaffected (data not shown). *De novo* repression of ICAT was induced by reexpression of the shRNA-insensitive preprogastrin cDNA (Figure 3A and B), as well as after treatment with 5 nM recombinant progastrin (data not shown).

In addition, ICAT expression was much weaker in the colonic epithelium of transgenic mice displaying tissue-specific intestinal overexpression of human progastrin (Tg/Tg) (Cobb et al., 2004) than that detected in their wild-type littermates (Figure 3C). Since the progastrin expressed by these mice is mutated and therefore unsensitive to processing enzymes (Cobb et al., 2004), this result demonstrates that full-length progastrin is also able to down-regulate ICAT expression *in vivo.*

Thus, endogenous progastrin is capable of repressing ICAT expression *in vitro* and *in vivo,* and progastrin depletion in CRC cells is sufficient to induce a strong *de novo* expression of this inhibitor.

Therefore, we investigated whether ICAT was indeed the molecular target involved in the regulation of β-catenin/Tcf-4 by progastrin in CRC cells. Using co-immunoprecipitation, we detected a significant increase in the association between dephosphorylated (B-catenin and ICAT in progastrin-depleted SW480/ *GAST*⁽⁻⁾ cells (Figure 4A), paralleled by the appearance of cytoplasmic colocalization of these two proteins (Figure 4B). In contrast, the amount of Tcf-4 co-immunoprecipitating with dephosphorylated β-catenin was significantly decreased compared to SW480/β-gal⁽⁻⁾ (Figure 4A). In turn, β-catenin was again preferentially bound to Tcf-4 after treatment of SW480/ *GAST*⁽⁻⁾ cells with recombinant progastrin, while binding to ICAT and colocalization of the two proteins were reduced (Figure 4A-B).

To confirm that ICAT re-expression in progastrin-depleted cells was functionally responsible for the inhibition of the β-cat/Tcf-4 transcriptional activity, we experimentally prevented the *de novo* expression of ICAT mRNA and protein in SW480/ *GAST*⁽⁻⁾ cells, using retrovirus-driven shRNA expression (Figure S4). We found that the activation of the β-catenin/Tcf-4 transcriptional complex was restored in the resulting SW480/ *GAST*⁽⁻⁾/ICAT⁽⁻⁾ cells (Figure 4C), which also displayed increased expression of Tcf-4 target genes (Figure 4D). In turn, this increase was prevented by overexpression of a codon-optimized version of ICAT cDNA in SW480/ *GAST*⁽⁻⁾/ICAT⁽⁻⁾ cells (Figure 4C, 4D and S4), demonstrating the specificity of ICAT in this process.

These results indicate that the modulation of ICAT expression levels is the major molecular event underlying the regulation Bcat/Tcf-4 activity by progastrin.

*In vivo,* low ICAT levels (Figure 5A and 5C) and high expression of Tcf-4 target genes (Figure 5B-C, and Figure S5) were detected in the progastrin-overexpressing intestinal tumors of APCΔ14 animals. In contrast, ICAT expression was significantly increased in adenomas collected from mice treated with *GAST*⁽⁻⁾-specific siRNAs (Figure 5A-C), concomitant with a strong repression of Tcf-4 target genes. Interestingly, expression of cyclin D1, c-Myc and CD44 in the macroscopically healthy epithelium of APC3,14/Luc⁽⁻) mice was found to be elevated when compared to *GAST*⁽⁻⁾ animals, suggesting that mutation of a single *Apc* allele is sufficient to partially increase target gene expression in APCΔ14 intestines, and that progastrin depletion is already effective to reduce Tcf-4 activity in this precancerous setting (Figure 5B and Figure S5).

In addition, levels of *GAST* mRNA in microdissected human colon tumors and matching healthy samples obtained from 23 patients with CRC (Figure S6) were increased to various degrees in the tumors of 78% of patients studied (18/23) (Figure 5D, top graph), while ICAT expression was down-regulated in the corresponding tumor samples (Figure 5D, bottom graph). A significant statistical correlation was found overall between increased *GAST* gene expression and repression of ICAT (Spearman's correlation coefficient: r = -0.46, p = 0.027), corroborating the relevance of the progastrin-induced repression of ICAT *in vivo* (Figure 5D, insert). This correlation was confirmed by immunohistochemistry on samples from patients with low (eg. patient 22) or high (eg. patient 4) progastrin expression, showing that ICAT was repressed and immunoreactivity for Tcf-4 target genes was strong in tumors expressing high progastrin levels, while these expression patterns were reversed in tumors with low progastrin (Figure 5E). Similar results were obtained on CRC samples from all 11 patients tested by immunohistochemistry, and this correlation mirrors the inverse correlation between ICAT levels and β-catenin/Tcf-4 activity observed *in vitro.*

Overall, these results demonstrate that, despite the constitutive activation of the β-catenin/Tcf-4 complex in *APC*-mutated cells, the activity of this pathway can be inhibited by progastrin depletion through a re-expression of the inhibitor ICAT, *in vivo* and *in vitro.* To our knowledge, these results also provide the first demonstration of an hormonal regulation of ICAT, a small inhibitor of β-catenin binding to Tcf-4 (Tago et al., 2000) previously described as a tumor suppressor (Daniels and Weis, 2002).

### Restoration of ICAT expression is responsible for the decreased tumorigenicity induced by progastrin depletion in CRC cells.

In view of the essential role played by the constitutive activation of β-catenin/Tcf-4 transcriptional activity in colon tumorigenesis, and since our results indicated that ICAT reexpression was responsible for the decreased Tcf-4-mediated transcription in progastrin-depleted tumor cells, we further investigated *in vitro* and *in vivo* the role of ICAT repression as an essential component of the tumor-promoting activity of progastrin.

*In vitro,* inhibition of ICAT re-expression in SW480/ *GAST*⁽⁻⁾/ICAT⁽⁻⁾ cells was found to enhance their capacity for anchorage-independent growth in a soft agar growth assay, up to a similar level to that found for SW480/β-Gal⁽⁻⁾cells (Figure 6A). *In vivo,* the ability of SW480/ *GAST*⁽⁻⁾/ICAT⁽⁻⁾ cells to form tumors after injection in BALB/c *nude* mice was much higher than that of SW480/ *GAST*⁽⁻⁾/Luc⁽⁻⁾ cells (Figure 6B), and similar to SW480/β-Gal⁽⁻⁾ cells (see Figure 1B).

Overall, these results clearly demonstrate that repression of ICAT by endogenous progastrin in CRC cells is instrumental to the tumor-promoting activity of this prohormone. Critically, they also show that the *de novo* expression of ICAT induced by progastrin depletion is strong enough to induce a significant inhibition of β-catenin/Tcf- 4 activity and thereby decrease tumor growth *in vitro* and *in vivo.*

### Phosphatidylinositol 3-kinase-mediated activation of Integrin-Linked Kinase is responsible for ICAT repression and modulation of the /3-catenin/Tcf-4 pathway by progastrin.

Since progastrin was recently shown to activate the phosphatidylinositol 3-kinase (PI3K) pathway in intestinal cells *in vitro* (Hollande et al., 2003) and *in vivo* (Ferrand et al., 2005), we determined whether activation of this enzyme was necessary for progastrin-induced ICAT repression and stimulation of β-catenin/Tcf-4 activity in SW480 cells.

First, we found that incubation of SW480/βgal⁽⁻⁾ cells with the selective PI3K inhibitor LY294002 was sufficient to induce the expression of ICAT (Figure 7A), and to reduce β-catenin/Tcf-4 transcriptional activity (Figure S7), thus unravelling a previously unknown link between these two pathways. In contrast, pharmacological inhibition of the Src or ERK1/2 pathway using 10 µM PP2 or 1 µM PD98059, respectively, did not affect ICAT expression or β-catenin/Tcf-4 activity (data not shown). In addition, LY294002 abolished the down-regulation of ICAT induced by a treatment of SW480/ *GAST*⁽⁻⁾ clones with recombinant progastrin (Figure 7A), showing that PI3K activation is essential to the repression of ICAT by progastrin. Accordingly, serine 473-phosphorylation of Akt/PKB, a downstream target of PI3K, decreased markedly in progastrin-depleted SW480/*GAST*⁽⁻⁾ cells, but was reinstated after incubation with 5nM recombinant progastrin (Figure 7A).

Ser473 of Akt/PKB is known to be a direct phosphorylation target of integrin-linked kinase (ILK) (Delcommenne et al., 1998). To determine whether ILK activation was the missing molecular link between progastrin and the repression of ICAT, we first assessed whether the expression and/or activity of ILK were regulated by progastrin in SW480 cells. Both the expression and activity of ILK were significantly decreased in SW480/ *GAST*⁽⁻⁾ cells, and this inhibition was partially reversed after treatment with 5nM recombinant progastrin (Figure 7B). These results are corroborated by data showing a drastic reduction in the serine phosphorylation of integrinβ1, a downstream target of ILK (Mulrooney et al., 2000) in SW480/ *GAST*⁽⁻⁾ clones (Figure 7B).

In addition, we found that transient expression of dominant-negative ILK (DN-ILK), which was confirmed to repress Akt/PKB phosphorylation, was able to induce ICAT expression in SW480/βgal⁽⁻⁾ cells (Figure 7C), indicating that this enzyme is involved in a signaling pathway controlling the expression of the ICAT gene. Pharmacological inhibition of PI3K in these SW480/βgal⁽⁻⁾/DN-ILK cells did not increase ICAT expression any further, indicating that the two enzymes are involved in the same pathway, or in redundant pathways, in the regulation of ICAT gene expression (data not shown).

Furthermore, the repression of ICAT mRNA and protein and the increased phosphorylation of Akt/PKB, induced by treatment of progastrin-depleted SW480/ *GAST*⁽⁻⁾ cells with recombinant progastrin, was prevented by dominant-negative-mediated inhibition of ILK (Figure 7C). In contrast, shRNA-mediated repression of ICAT in SW480/ *GAST*⁽⁻⁾ cells (as shown in Figure S4) did not alter ILK expression or activity (data not shown), confirming that ICAT is located downstream of ILK in progastrin-mediated signaling.

These results clearly demonstrate that the activations of PI3K and ILK are instrumental to the progastrin-mediated repression of ICAT in CRC cells.

### Depletion of progastrin in vitro and in vivo induces the terminal differentiation of intestinal tumor cells along the mucus-producing goblet cell lineage.

Since the Wnt/B-catenin/Tcf-4 pathway is deemed to be essential for the maintenance of the stem/progenitor phenotype of cells at the bottom of the intestinal crypt, and is widely seen as a molecular switch between cell proliferation and differentiation (Radtke and Clevers, 2005; van de Wetering et al., 2002), we next determined whether its down-regulation in progastrin-depleted CRC cells was able to drive these cells towards differentiation.

In view of its recently described role as an early promoter of cell differentiation (Chung and Eng, 2005), we first analyzed the localization of PTEN (phosphatase and tensin homologue deleted from chromosome 10) in stable SW480/ *GAST*⁽⁻⁾ clones. In striking contrast with its predominantly cytoplasmic localization in SW480/βgal⁽⁻⁾ cells, PTEN was mostly found in the nucleus of SW480/ *GAST*⁽⁻⁾ clones (Figure 8A), a localization thought to be associated with its role in cell differentiation (Lian and Di Cristofano, 2005).

Since the process of developing stable progastrin-depleted clones is expected to select the least differentiated and non-apoptotic cells, the expression of genes associated with terminally differentiated intestinal cell lineages was quantified in SW480 cells, after transient transfection with rhodamine-tagged siRNA oligonucleotides directed against the *GAST* or the P-galactosidase gene. A significant induction in the expression of the goblet cell-specific gene Muc-2 was detected in SW480/ *GAST*⁽⁻⁾ but not in SW480/β-gal⁽⁻⁾ cells, without detectable alterations of the genes encoding chromogranin A (expressed in enteroendocrine cells) or enterocyte-specific alkaline phosphatase (Figure 8B). Finally, the detection of caspase-3 activation in *GAST*⁽⁻⁾ cells, and the finding that cells positive for activated caspase-3 were also Muc2-positive (Figure 8C), indicated that progastrin depletion was capable of differentiating CRC cells towards a secretory lineage, before promoting their apoptosis *in vitro.* Treatment with recombinant progastrin for 48h reversed these phenotypes (Figure 8C), while amidated and glycine-extended gastrins had no effect (not shown). Similar results were obtained with DLD-1 cells transfected with GAST-specific siRNA oligonucleotides (not shown). Thus progastrin-depletion was capable of driving CRC cells towards a terminal differentiation and apoptosis *in vitro.*

In addition, we sought to demonstrate that reduction of progastrin levels *in vivo* would also be sufficient to induce differentiation of intestinal tumors induced by a constitutive activation of the β-catenin/Tcf-4 pathway. Immunohistochemistry on samples collected from APCΔ14 mice treated with luciferase or *GAST*-specific siRNAs (see Figure 1), demonstrated that the remaining small intestinal and colonic adenomas of *APCΔ*14/ *GAST*^{(-)*)*} animals displayed greatly increased numbers of Muc-2 and Alcian-blue positive cells compared to controls (Figure 8D), in line with the increased expression of ICAT and the reduced expression of c-Myc in the same samples (see Figure 5A-C). Finally, while cells staining for activated caspase 3 were very scarce in control animals, their numbers were found to be strongly elevated in adenomas from APCΔ14/ *GAST*⁽⁻⁾ animals (Figure 8D). These results demonstrate that siRNA-mediated down-regulation of the *GAST* gene is able to drive tumor cells towards terminal differentiation and apoptosis *in vivo.* Intestinal adenomas developed by APCΔ14 mice do not express glycine-extended or amidated forms of gastrin. Since high molecular weight processing intermediates were never detected by immunoblotting using polyclonal antibodies raised against recombinant progastrin, and since full-length recombinant progastrin was shown to regulate CRC cell differentiation *in vitro,* we conclude that tumor cell differentiation and reduced tumor growth after GAST-specific siRNA treatment is mostly due to reduced concentrations of full-length progastrin.

### DISCUSSION

The present work provides the demonstration that blocking the production of progastrin significantly inhibits "constitutive: beta-catenin/Tcf-4 transcriptional activity in human CRC cells, reduces their anchorage-independent growth *in vitro* as well as their ability to form tumors in *nude* mice, and promotes their differentiation and apoptosis. Progastrin depletion resulted in re-expression of the "Inhibitor of beta-catenin And Tcf-4" (ICAT), a small peptide originally identified in *Xenopus* and shown to inhibit the interaction of beta-catenin with Tcf-4 (Gottardi and Gumbiner, 2004; Tago et al., 2000). In addition, treatment with recombinant progastrin down-regulated ICAT and increased beta-catenin/Tcf-4 activity in cells where GAS gene expression had previously been switched off, confirming that chronic progastrin secretion was involved in maintaining a high level of activation for this pathway in CRC cell fines. The physiological relevance of the link unraveled here between chronic progastrin secretion and ICAT repression was emphasized *in vivo* by results showing a tight correlation between these two events within colorectal tumors in a panel of 16 patients with CRC, and by the demonstration that ICAT expression was down-regulated in the colonic epithelium of tumor-susceptible, progastrin-overexpressing mice (Cobb et al., 2004).

This data is consistent with the previous observation that progastrin promotes the development of colorectal tumors and provides a molecular explanation for the strong reduction in the number and size of adenomas observed in gastrin-deficient APCmin^{+/-} mice (Koh et al., 2000). Furthermore, expression profiling of target genes in gastric parietal cells from mice with a null-mutation in the GAS gene compared to wild-type mice from a similar background, showed that 20% of differentially expressed genes were also known to be target genes for Wnt and Myc, emphasizing the link between these signaling pathways (Jain et al., 2006).

It is of note that, while increased progastrin expression in CRC is well documented (Ciccotosto et al., 1995; Konturek et al., 2002; Siddheshwar et al., 2001; Van Solinge et al., 1993), the repression of ICAT described in the present work conflicts with the only previous report concerning this field (Koyama et al., 2002). This discrepancy could be due to the fact that the increased expression of ICAT reported by Koyama et *al*. was measured using semi-quantitative RT-PCR on non-microdissected tissue. Our results, allowing for a direct comparison of ICAT expression by cells of epithelial origin in the healthy mucosa and in tumors, appear to be more consistent with the described role for ICAT as a tumor suppressor. Indeed, progastrin-mediated ICAT repression appears essential for tumor growth, since newly synthesized ICAT in progastrin-depleted CRC cells was shown to compete with Tcf-4 for binding to beta-catenin, resulting in decreased transcriptional activity of Tcf-4 and decreased tumorigenicity, in agreement with previously reported data concerning this peptide (Sekiya et al., 2002).

The capacity to reinstate, albeit incompletely, the repression of ICAT and the activation of the beta-catenin/Tcf-4 pathway by treatment with exogenous progastrin, argues in favor of the existence of an autocrine/paracrine loop mediated by a membrane receptor. The analysis of signaling events involved in ICAT-mediated modulation of beta-catenin/Tcf-4 activity by progastrin allowed us to identify a novel link between the beta-catenin/Tcf-4 and Pl3Kinase/ILK pathways in CRC cells. Indeed, pharmacological inhibition of PI3K and dominant-negative induced disruption of ILK signaling increased ICAT expression and prevented progastrin-mediated repression of this peptide. Previous results by Tan et *al*. (Tan et al., 2001) demonstrated that inhibition of ILK suppressed beta-catenin-Tcf/Lef-dependent transcription through repression of snail, thereby increasing E-cadherin expression and recruitment of beta-catenin at the membrane in APC-mutated human colon carcinoma cells. Results from the present work indicate that even in non-confluent cells, where membrane-recruitment of beta-catenin is low, progastrin-mediated ILK signaling is able to modulate beta-catenin/Tcf-4 activity through a control of ICAT expression.

Finally, our results also show that agents antagonizing progastrin functions provide new therapeutic options to support the present surgical approach in colorectal cancer. Results presented here show that inhibition of progastrin signaling provides a specific therapeutic alternative for colon cancer management. Since progastrin seems to be secreted only by the tumor and not by the healthy epithelium, such a strategy should counteract the effect of the frequent APC mutations and decrease the activation of beta-catenin/Tcf-4 signaling closer to physiological levels, thereby promoting the preservation of normal crypt integrity.

### METHODS

### Cells and cell culture conditions

Colorectal cancer cell fines DLD1 and SW480 were maintained at 37°C in Dulbecco's Modified Eagle Medium (DMEM) supplemented with 10% fetal bovine serum (Eurobio, Les Ulis, France), 1% L-glutamine and penicillin/streptomycin.

### Materials

The following primary antibodies were used: mouse anti-dephosphorylated beta-catenin (clone 8E4; AG. Scientific), mouse anti beta-catenin and anti-E-cadherin (Transduction Laboratories), goat anti-Tcf-4 (Santa Cruz Biotechnology), rabbit anti-ICAT (a generous gift from Dr C. Gottardi; (Gottardi and Gumbiner, 2004)), polyclonal anti-PTEN (Upstate), rabbit anti-phosphoserin (Zymed), rabbit anti-ILK for western blot (Cell Signaling) and for immunoprecipitation (Upstate), mouse anti-integrin β1 (Chemicon), rabbit anti-Akt, anti-Ser473-phosphorylated Akt, anti-activated caspase-3 (Cell Signaling), Anti-Muc2 monoclonal antibody (Neomarkers),

### Human tissue collection

Specimens of colon tumors and histologically normal epithelium (taken at a significant distance from the tumor) from 16 patients, were obtained from the pathologist after resection according to French government regulations and local committee guidelines. Informed consent was obtained from all patients. Tissue samples were stored in liquid nitrogen until further use.

### Tissue sections, microdissection and RNA preparation

Tissue sections were prepared from liquid nitrogen-frozen tumor samples, and hematoxylin/eosin staining was performed to assess their quality, orientation within the tissue, and epithelial content. Immunofluorescent detection of beta-catenin, Tcf-4, c-myc and ICAT was performed on sections adjacent to those used for microdissection.

Laser Capture Microdissection (LCM) was performed on four to six sections per sample using a PixCell® Ile microdissector from Arcturus (Alphelys, Plaisir, France). The following laser beam settings were used: 265mV, 45mWh, 15fam diameter, 18ms. Microdissected tissues were then directly collected in RNA lysis buffer and RNA was prepared using the RNAeasy Microkit (Qiagen, Courtaboeuf, France), according to the manufacturer's instructions. The quality and amount of RNA recovered were assessed using RNA pico Labchips (Agitent Technologies, Palo Alto, California).

### Stable transfection

Transfection of gastrin gene antisense and control cDNA into DLD1 cell lines was described in (Hollande et al., 2003). In order to generate progastrin shRNA, the following oligonucleotides were cloned into pSilencer vector (Ambion) and transfected in SW480 cells: sense: 5'-GAAGAAGCCTATGGATGGATTCAAGAGAAGGTAGGTATCCGAAGAAGTTTTTT-3' (SEQ ID N°1) and antisense: 5'-AATTAAAAAACTTCTTCGGATACCTACCTTCTCTTGAATCCATCCATAGGCTT CTTCGGCC 3' (SEQ ID N°2). β-galactosidase oligonucleotides used to generate control shRNA were: sense: 5' GATCCCAAGGCCAGACGCGAATTATTTTTCAAGAGAAAATAATTCGCGTCTGGCCTTTTTTTG GAAA-3' (SEQ ID N°3); antisense: 5'-AGCTTTTCCAAAAAAAGGCCAGACGCGAATTATTTTCTCTTGAAAAATAATTCGCGTCTGGCCT TGG-3' (SEQ ID N°4). 5 X 10⁴ cells/well were seeded and transfected 24h later with 500 ng of pSilencer/progastrin-shRNA or pSilencer/β-galactosidase-shRNA, 50 ng of pCDNA-3, and 5 µl/well of Exgen 500 (Euromedex). Selection was performed with Neomycin (500ng/p.1).

Retrovirus-mediated transfection of ICAT or Luciferase shRNA in SW480/*GAS*⁽⁻⁾ cells shRNA oligonucleotides for ICAT sense 5' GATCCGGATGGGATCAAACCTGACATTTTCAAGAGAAATGTCAGGTTTGATC CCATCTTTTTTG 3' (SEQ ID N°5) and antisense 5' AATTCAAAAAAGATGGGATCAAACCTGACATTTCTCTTGAAAATGTCAGGTTTGATCCCATCCG 3' (SEQ ID N°6). and luciferase (sense 5'GATCCGACATTAAGAAGGGCCCAGCTTTTCAAGAGAGCTGGGCCTTAATCTTTTTT 3' (SEQID N°7), and antisense S'AATTGATTAAGGCCCAGCTCTCTTGAAAAGCTGGGCCCTTCTTAATGTCG 3' (SEQ ID N°8) were cloned into pSIREN-retroQ (Clontech). The amphotropic packaging cell line (ΦNX; Dr Garry Nolan, Stanford University, Stanford, California) was transfected as described by Pear et al (Pear et al., 1993). The medium containing the virus was removed 48 hr after transfection and centrifuged at 1500 rpm for 5 min to pellet cell debris. Target cells (2.10⁵/well in 6 well-plates) were infected in the presence of polybrene (8 µg/ml) with 2 ml of the centrifuged medium containing the virus. Infected cells were treated with puromycin (5 µg/ml) in order to select for positive clones.

### Transient transfections

The day before transfection, SW480 cells were seeded in 24-well plates (5x 10⁴ cells/well). Cells were transfected with 100 pmol of rhodamine tagged *GAS*-specific or β-galactosidase-specific shRNA, and 5 µl/well of Exgen 500 (Euromedex), according to the manufacturer's instructions. 48 hr or 72 hr after transfection, cells were lysed (for immunoblotting and PCR) or fixed in 1 % paraformaldehyde for immunostaining.

### Alcian blue staining

72 hr after transfection, cells on coverslips were washed with PBS and incubated for 30 min with alcian blue (10g/l in 3% acetic acid). After rinsing cells in water, coverslips were mounted on glass slides in mowiol and stored at 4°C until use.

### RT-Quantitative PCR

2.5 µg of total RNA was pretreated with DNAse RQ1 (Promega) for 30 min at 37°C and 25 used for reverse transcription with M-MLV reverse transcriptase *(Invitrogen).* Quantitative PCR was carried out using the LightCycler FastStart DNA MasterPlus SYBR Green I kit (Roche Diagnostics, Meylan, France), under the following conditions:
Actin: denaturation during 10 min at 95°C, amplification for 50 cycles: 10 sec at 95°C, 6 sec at 58°C, 11 sec at 72°C. Melting curve: 0 sec at 95°C, 30 sec at 68°C, 0 sec at 95°C and cooling was for 2 min at 40°C: c-myc, ILK, chromogranin A and GAPDH amplification: denaturation during 10 min at 95°C, amplification for 50 cycles: 10 sec at 70°C, 6 sec at 58°C, 13 sec at 72°C. The melting curve was 0 sec at 95°C, 30 sec at 80°C, 0 sec at 95°C and cooling was for 2 min at 40°C. Progastrin, Muc-2, ALP and ICAT amplification: denaturation during 10 min at 95°C, amplification during 50 cycles with: 10 sec at 70°C, 6 sec at 95°C, 11 sec at 72°C. The melting curve was 0 sec at 95°C, 30 sec at 80°C, 0 sec at 70°C and cooling was for 2 min at 40°C. Primers used for amplification of selected genes were: GAPDH-sense: 5'GGTGGTCTCCTCTGACTTCAACA3' (SEQ ID N°9) antisense: 5'GTTGCTGTAGCCAAATTCGTTGT3' (SEQ ID N°10); Actin-sense: 5'CGGGAATCTGCGTGACAT3' (SEQ ID N°11); antisense:5 'AAGGAAGGCTGGAAGAGTGC3' (SEQ ID N°12); ILK-sense: 5'ATGACTGCCCGAATTAGCATG3' (SEQ ID N°13); antisense: 5'GCTACCCAGGCAGGTGCATA3' (SEQ ID N°14); ICAT-sense: 5'GCTCTGGTGCTTTAGTTAGG3' (SEQ ID N°15); antisense: 5'GCACTTGGTTTCTTTCTTTTC3' (SEQ ID N°16); c-myc-sense: 5'CGTCTCCACACATCAGAGCACAA3' (SEQ ID N°17); antisense: 5'TCTTGGCAGCAGGATAGTCCTT3' (SEQ ID N°18); human progastrin-sense: 5'AGAGGATCCAAATGCAGCGACTATGTGTGTATG3' (SEQ ID N°19); antisense: 5'GGCGAATTCCTAGGATTGTTAGTTCTCATCCTCAG3' (SEQ ID N°20); Muc-2-sense: 5'TGGGTGTCCCTCGTCTCCTACA3' (SEQ ID N°21); antisense: 5'TGTTGCCAAACCGGTGGTA3' (SEQ ID N°22); Alkaline Phosphatase-sense: 5'CTCCAACATGGACATTGACG3' (SEQ ID N°23); antisense: 5'CAGTGCGGTTCCACACATAC3' (SEQ ID N°24); Chromogranin A-sense: 5'ATCACCGCCACTGCCACCACCA3' (SEQ ID N°25); antisense: 5'CACCTTAGTGTCCCCTTTTGTCATAGGGCT3' (SEQ ID N°26).

### Soft agar colony formation assay

1 X 10⁵ cells were resuspended in 0.2% agar in DMEM-F12 supplemented with 10% fetal bovine serum, 1% glutamine and penicillin/streptomycin, and plated onto a 6-cm Petri dish containing a solidified bottom layer (0.5% agar in growth medium). 8 days after plating, cells were stained in 0.02% crystal violet and the size and number of colonies was quantified under a microscope. All clones were seeded in triplicates and ten fields were counted for each.

### In vivo studies

*In vivo* experiments were performed following the French guidelines for experimental animal studies (Direction des Services Vétérinaires, Ministère de l'Agriculture, Agreement N° B34-172-27) and fulfill the UKCCCR guidelines for the welfare of animals in experimental neoplasia. 2 X 10⁶ cells were injected subcutaneously into 6-week-old athymic BALB/c-nu/nu *(nude)* mice. Tumor growth was followed regularly thereafter (Estimated tumor volume = (Length x width x thickness)/2).

APCΔ14 mice were housed in conventional conditions and randomized in two groups. The first group (n=9), was treated once daily by intraperitoneal administration of 250 µg/kg siRNA against the murine *GAST* mRNA. Control mice (n=9) were treated with a similar dose of siRNA against Luciferase. (siRNA sequence on Figure S7 online). SiRNA design incorporated 3' overhangs and did not contain a 5'UGUGU3' sequence, to minimize proinflammatory response and degradation *in vivo* (Judge et al., 2005; Marques et al., 2006). After a 10-day treatment, mice were culled, their blood collected and their intestine dissected out. Adenoma scoring in the intestine and colon was described in (Colnot et al., 2004). Samples of healthy intestinal mucosa, as well as intestinal and colon adenomas, were fixed in 4% PFA and paraffin-embedded, or frozen in liquid nitrogen, for Western blotting, IHC or RNA extraction. Plasma was prepared and assayed for IL-6 and TNFα using the FACSarray system (Becton Dickinson).

### Luciferase reporter gene assay

The protocol used to measure the transcriptional activity of TCF/LEF-1 was described before (Morin et al., 1997). Briefly, cells were transfected with 500 ng of TCF/LEF-1 reporter (pTOP-FLASH), or control vector (pFOP-FLASH) and with 25 ng pCMV-Renilla. Luciferase activity was quantified using the Dual luciferase reporter assay system (Promega), and was normalized relative to the activity of Renilla.

### Immunofluorescent staining

Immunofluorescent staining was performed as described previously (Hollande et al., 2003). Slides were then observed using a laser scanning LEICA sp2 confocal microscope with a x40 oil immersion lens.

### Immunochemistry on paraffin tissue sections

Paraffin tissue sections were prepared from FVB/N mice that were either transgenic for the human GAS gene (Tg/Tg) or wild type littermates (Cobb et al., 2004). Following dewaxing and hydration, sections were pretreated with peroxidase for 20 min at room temperature. Antigen was retrieved by boiling samples for 20 min in 10mM Tris - 1mM EDTA, pH 9. Slides were allowed to cool down to room temperature and incubated overnight at 4°C with antibodies in PBS +0.05% BSA. In all cases, the Envision+ kit (DAKO) was used as a secondary reagent. Stainings were developed using DAB (Sigma) and slides were counterstained with either hematoxylin or Nuclear Fast Red and mounted.

### Protein lysates, immunoprecipitation, SDS-PAGE and Western immunoblotting

Protein lysates, immunoprecipitations and immunoblotting were performed as described before (Hollande et al., 2003) Proteins were visualized using ECL Plus (Amersham Biosciences) and the bands were quantified by densitometry using ImageJ 1.32J (NIH, Bethesda, MD).

### Immune complex kinase assays

The reaction was started by the addition of 25 µl of kinase buffer (50 mM Hepes pH 7.0, 1mM MnCl₂, 1 mM Na orthovanadate, 2 mM NaF, 5 µg Myelin Basic Protein (Sigma)) with 0.5 µg of ATP (250 mM ATP, 1 µCi[γ-³²P]ATP) per tube, and incubated at 30°C for 20 min. The reaction was terminated with the addition of 10 µl of 4X sample buffer. The tubes were centrifuged at 10,000g for 1 min and the proteins were resolved on a 14% SDS-page gel. Phosphorylation of the substrate was visualized by autoradiography (Marotta et al., 2003).

### Radioimmunoassay

GAS gene products progastrin, glycine-extended gastrin (G-gly) and amidated gastrin (G-NH2) in cell supernatants were quantitated by radioimmunoassay, as described before (Hollande et al., 1997).

### Statistical Analysis

All statistical analyses were performed using SAS version 9.1 for Windows (Cary, NC, USA). Student's t-test was used in order to determine the statistical significance of differences between control and progastrin-depleted cells, with or without treatment with recombinant progastrin. In order to determine the association between GAS and ICAT gene expressions in human tumors, the variables were normalized using their natural log values before determining Pearson's correlation coefficient (r).

### Example 2

### Selective antibodies against progastrin reverse the repression of progastrin on ICAT expression and induce a decrease in c-myc expression.

### INTRODUCTION

The present example describes the characterization of two independently generated polyclonal antibodies against progastrin, aiming to demonstrate that they selectively recognize the full-length progastrin (1-80) peptide but not the processed peptides potentially present within human blood, such as amidated gastrin, glycine-extended gastrin, and the six amino-acid C-terminal flanking peptide (CFTP). This characterization was performed in vitro using an ELISA assay.

The second step described hereunder was the validation of the proof of concept that the effects of progastrin on ICAT expression and on beta-catenin/Tcf4 activity can be blocked by a selective antibody. In order to demonstrate this so-called neutralizing activity of the progastrin antibodies, we used human SW480 colorectal tumor cells, which bear a mutation in the *apc* gene and display endogenous progastrin secretion due to the constitutive beta-catenin / Tcf4 activity. The capacity of these cells to secrete high amounts of progastrin and very low levels of amidated and glycine-extended gastrin is described in Table I.

These cells were treated independently with two different polyclonal antibodies, one directed against the N-terminal sequence of progastrin, the other against the C-Terminal sequence.
The N-Terminal sequence was: SWKPRSQQPDAPLGT (SEQ ID N°32)
The C-terminal sequence was: FGRRSAEDEN (SEQ ID N°31)

### RESULTS

- Antibody selectivity: Selectivity of antibodies was assessed by an ELISA test described in the Methods section. Results (Figure 10) show that both polyclonal antibodies selectively recognize progastrin and the respective peptides used as immunogens for their production. They do not bind to Glycine-extended Gastrin, to amidated gastrin or to the C-terminus flanking peptide (CTFP) (sequence: SAEDEN), another peptide derived from the maturation process of progastrin and detected in the human serum (Smith KA, Gastroenterology 2006).
- Neutralizing activity of progastrin antibodies: The ability of progastrin antibodies to inhibit the repression of ICAT by progastrin and to induce a resulting decrease in the transcriptional activity of the beta-catenin/Tcf4 complex was addressed in SW480 colorectal carcinoma cells. When cells were incubated for 30 hours in the presence of rabbit polyclonal antibodies directed against the N-terminal or C-terminal extremity of progastrin(1-80), both at a 1/5000 dilution, ICAT mRNA expression was strongly increased while those of c-Myc and cyclin D 1 were markedly decreased, in comparison with cells treated with the same concentration of a non-specific rabbit polyclonal antibody. C-myc and cyclin D1 are recognized targets of beta-catenin / Tcf4 transcriptional activity, and the decrease in their expression is considered as a consequence of the decrease in beta-catenin/Tcf4 activity (Figure 11).

### CONCLUSION

These results demonstrate in vitro that progastrin inhibition of ICAT expression can be reversed by its binding to a selective antibody, resulting in an inhibition of the constitutive beta-catenin / Tcf4 activity. It is thus provided herein for the first time the proof of concept that a progastrin antibody can be used to block the protumorigenic effects of progastrin occurring via ICAT and beta-catenin / Tcf4 activity.

### METHODS

### ELISA

Antigens were diluted at the indicated concentration in PBS, and 100 µl were coated in multisorb 96 well plates at 4°C overnight. The next day, wells are washed 3 times with 200 µl of PBS/Tween 1% and 100 µl of the blocking solution (PBS/Tween 1%/BSA 0.1%) was added for 2 hours at 22°C. After another 3 washes with PBS/1% Tween, primary antibodies were diluted in the blocking solution and 100 µl was added to the wells for 2 hours at 22°C. Antibody concentration is indicated in the Figures. Secondary antibodies are diluted in the same blocking buffer and after 3 washes, 100 µl are added to the wells for 2 hours at 22°C. Finally, wells were washed again with PBS/Tween 1% and 100 µl of OPD solution substrate was added for 20 minutes at 22°C. The reaction was stopped with 50 µl of H₂SO₄ 4N and reading was performed at 492 nm.

### Cell culture and treatment with progastrin-selective antibodies

The CRC cell line SW480 was maintained at 37°C in Dulbecco's Modified Eagle Medium (DMEM) supplemented with 10 % fetal bovine serum (Eurobio, Les Ulis, France), 1 % L-glutamine and penicillin/streptomycin. SW480 cells were plated in 6 well plates (200,000 cells/well) in DMEM containing 10% FBS, 1% antibiotics and 1% glutamine, left to grow overnight at 37°C, 5%CO₂, then serum starved for 24 hours. The next morning, the medium was replaced with DMEM without FBS containing a 1/5,000 dilution of control or progastrin-selective polyclonal antibodies, followed by incubation at 37°C, 5%CO₂. The medium containing antibodies was renewed 12 hours later. After 30 hours, cells were washed with PBS and lysed with the RNA extraction kit lysis buffer.

### RNA preparation and RT-Quantitative PCR

RNA was prepared from SW480 cells using the RNeasy Protect Minikit (Qiagen France SA, 91974 Courtaboeuf, France). The quality and amount of RNA recovered were assessed using RNA pico Labchips (Agilent Technologies, Palo Alto, California). For reverse transcription, 2.5 µg of total RNA from each sample was pretreated with DNAse RQ1 (Promega) for 30 min at 37°C and incubated with M-MLV (InVitrogen). Quantitative PCR was carried out from 24 of cDNA per sample, using the LightCycler FastStart DNA MasterPlus SYBR Green I kit (Roche Diagnostics). Expression of GAPDH mRNA was used to calibrate RNA loading.

### Cycle conditions for Quantitative PCR

- For c-myc and GAPDH amplification: denaturation during 10 min at 95°C, amplification for 50 cycles: 10 sec at 95°C, 6 sec at 58°C, 13 sec at 72°C. The melting curve was 0 sec at 95°C, 30 sec at 80°C, 0 sec at 95°C and cooling was for 2 min at 40C°.
- For ICAT amplification: denaturation during 10 min at 95°C, amplification during 50 cycles with: 10 sec at 95°C, 6 sec at 60°C, 11 sec at 72°C. The melting curve was 0 sec at 95°C, 30 sec at 80°C, 0 sec at 70°C and cooling was for 2 min at 40°C.
- For cyclin D1 amplification: 10 min at 95°C, amplification during 50 cycles with: 10 sec at 95°C, 6 sec at 70°C, 11 sec at 72°C. The melting curve was 0 sec at 95°C, 30 sec at 80°C, 0 sec at 95°C and cooling was for 2 min at 40°C.

### Primers for Quantitative PCR on human sequences:

GAPDH-sense: 5'GGTGGTCTCCTCTGACTTCAACA3' (SEQ ID N°33)
GAPDH-antisense: 5'GTTGCTGTAGCCAAATTCGTTGT3' (SEQ ID N°34)
ICAT-sense: 5'GCTCTGGTGCTTTAGTTAGG3' (SEQ ID N°35)
ICAT-antisense: 5'GCACTTGGTTTCTTTCTTTTC3' (SEQ ID N°36)
c-Myc-sense: 5'CGTCTCCACACATCAGAGCACAA3' (SEQ ID N°37)
c-Myc-antisense: 5'TCTTGGCAGCAGGATAGTCCTT3' (SEQ ID N°38)
Cyclin D1-sense: 5'-CCGTCCATGGGGAAGATC-3' (SEQ ID N°39)
Cyclin D1-antisense: 5'-ATGGCCAGCGGGAAGAC-3' (SEQ ID N°40)

### GENERAL CONCLUSIONS ON EXAMPLES 1 AND 2

For the first time, the sufficient and necessary data are provided demonstrating that progastrin depletion of human colorectal tumor cells reverses tumorigenesis, as it induces differentiation and apoptosis of cells that have a constitutive beta-catenin / Tcf4 activity. These effects are specific of progastrin. This is shown *in vitro* on two cell fines (DLD-1 and SW480) as well as *in vivo* with these same cell fines engrafted in nude mice and in a mouse model recapitulating the human tumorigenesis. Indeed, in this mouse model (APCΔ14), the *apc* gene bears a mutation leading to activation of the beta-catenin / Tcf4 pathway and mice spontaneously develop adenomas and adenocarcinomas. These mice were treated *in vivo* with a siRNA targeting the peprogastrin mRNA. This resulted in a reversal of tumorigenesis, with differentiation and apoptosis leading to tumor shrinkage. It is shown that this treatment profoundly inhibits the constitutive beta-catenin / Tcf4 activity. Therefore, it is the very first time that tumorigenesis initiated by an *apc* gene mutation is shown to be reversed. At the molecular level, it is shown that depletion of progastrin has anti-tumor effects because it induces the re-expression of ICAT, an endogenous inhibitor of beta-catenin/Tcf4 transcriptional activity.

In human colorectal cancer, we demonstrate for the first time the occurrence of a correlation between hyperactivation of the beta-catenin / Tcf4 pathway, high levels of gastrin gene expression and low levels of ICAT expression. In addition, over expression of progastrin itself was shown in tumor samples where Tcf4 target gene levels are elevated. In spontaneously arising intestinal adenomas of APCΔ14 mice, we also detected high levels of progastrin (and not of glycine-extended gastrin and amidated gastrin) and low ICAT expression. These parameters were reversed after treatment with gastrin gene selective siRNA. Finally, data are provided demonstrating that blocking the effects of progastrin can also be achieved with antibodies specifically directed against progastrin, unable to bind glycine-extended gastrin or gastrin.

Proofs of concept and sufficient scientific data are thus provided for the use of either siRNA, shRNA or antibodies targeting progastrin to induce expression of ICAT and block and reverse colon tumorigenesis associated with a constitutive beta-catenin / Tcf4 activity.

### References:

Blache, P., M. van de Wetering, I. Duluc, C. Domon, P. Berta, J.N. Freund, H. Clevers, and P. Jay. 2004. SOX9 is an intestine crypt transcription factor, is regulated by the Wnt pathway, and represses the CDX2 and MUC2 genes. J Cell Mol. 166:37-47.
Brown, D., U. Yallampalli, A. Owlia, and P. Singh. 2003. pp60c-Src Kinase mediates growth effects of the full-length precursor progastrin1-80 peptide on rat intestinal epithelial cells, in vitro. Endocrinology. 144:201-11.
Chakladar, A., A. Dubeykovskiy, L.J. Wojtulciewicz, J. Pratap, S. Lei, and T.C. Wang. 2005. Synergistic activation of the murine gastrin promoter by oncogenic Ras and beta-catenin involves SMAD recruitment. Biochem Biophys Res Commun. 336:190-6.
Chung, J.H., and C. Eng. 2005. Nuclear-cytoplasmic partitioning of phosphatase and tensin homologue deleted on chromosome 10 (PTEN) differentially regulates the cell cycle and apoptosis. Cancer Res. 65:8096-100.
Ciccotosto, G.D., A. McLeish, K.J. Hardy, and A. Shulkes. 1995. Expression, processing, and secretion of gastrin in patients with colorectal carcinoma. Gastroenterology. 109:1142-53.
Cobb, S., T. Wood, J. Ceci, A. Varro, M. Velasco, and P. Singh. 2004. Intestinal expression of mutant and wild-type progastrin significantly increases colon carcinogenesis in response to azoxymethane in transgenic mice. Cancer. 100:1311-23.
Cobb, S., T. Wood, L. Tessarollo, M. Velasco, R. Given, A. Varro, N. Tarasova, and P. Singh. 2002. Deletion of functional gastrin gene markedly increases colon carcinogenesis in response to azoxymethane in mice. Gastroenterology. 123:516-30.
Coelho, S.M., M. Vaisman, and D.P. Carvalho. 2005. Tumour re-differentiation effect of retinoic acid: a novel therapeutic approach for advanced thyroid cancer. Curr Pharm Des. 11:2525-31.
Colnot, S., M. Niwa-Kawakita, G. Hamard, C. Godard, S. Le Plenier, C. Houbron, B. Romagnolo, D. Berrebi, M. Giovannini, and C. Perret. 2004. Colorectal cancers in a new mouse model of familial adenomatous polyposis: influence of genetic and environmental modifiers. Lab Invest. 84:1619-30.
Daniels, D.L., and W.I. Weis. 2002. ICAT inhibas beta-catenin binding to Tcf/Lef-family transcription factors and the general coactivator p300 using independent structural modules. Mol Cell. 10:573-84.
Delcommenne, M., C. Tan, V. Gray, L. Rue, J. Woodgett, and S. Dedhar. 1998. Phosphoinositide-3-OH kinase-dependent regulation of glycogen synthase kinase 3 and protein kinase B/AKT by the integrin-linked kinase. Proc Nad Acad Sci USA. 95:11211-6.
Ferrand, A., C. Bertrand, G. Portolan, G. Cui, J. Carlson, L. Pradayrol, D. Fourmy, M. Dufresne, T.C. Wang, and C. Seva. 2005. Signaling pathways associated with colonic mucosa hyperproliferation in mice overexpressing gastrin precursors. Cancer Res. 65:2770-7.
Finley, G.G., R.A. Koski, M.F. Melhem, J.M. Pipas, and A.I. Meisler. 1993. Expression of the gastrin gene in the normal human colon and colorectal adenocarcinoma. Cancer Res. 53:2919-26.
Fodde, R., R. Smits, and H. Clevers. 2001. APC, signal transduction and genetic instability in colorectal cancer. Nat Rev Cancer. 1:55-67.
Gottardi, C.J., and B.M. Gumbiner. 2004. Role for ICAT in beta-catenin-dependent nuclear signaling and cadherin functions. Am J Physiol Cell Physiol. 286:C747-56.
Hollande, F., A. Imdahl, T. Mantamadiotis, G.D. Ciccotosto, A. Shulkes, and G.S. Baldwin. 1997. Glycine-extended gastrin acts as an autocrine growth factor in a nontransformed colon cell line. Gastroenterology. 113:1576-88.
Hollande, F., D.J. Lee, A. Choquet, S. Roche, and G.S. Baldwin. 2003. Adherensjunctions and tight junctions are regulated via different pathways by progastrin in epithelial cells. J Cell Sci. 116:1187-97.
Hollande, F., A. Shulkes, and G.S. Baldwin. 2005. Signaling the junctions in gut epithelium. Sci STKE. 2005:pe13.
Jain, R.N., C.S. Brunkan, C.S. Chew, and L.C. Samuelson. 2006. Gene expression profiling of gastrin target genes in parietal cells. Physiol Genomics. 24:124-32.
Janssen, K.P., P. Alberici, H. Fsihi, C. Gaspar, C. Breukel, P. Franken, C. Rosty, M. Abal, F. El Marjou, R. Smits, D. Louvard, R. Fodde, and S. Robine. 2006. APC and Oncogenic KRAS Are Synergistic in Enhancing Wnt Signaling in Intestinal Tumor Formation and Progression. Gastroenterology. 131:1096-109.
Judge, A.D., V. Sood, J.R. Shaw, D. Fang, K. McClintock, and I. MacLachlan. 2005. Sequence-dependent stimulation of the mammalian innate immune response by synthetic siRNA. Nat Biotechnol. 23:457-62.
Kochman, M.L., J. DelValle, C.J. Dickinson, and C.R. Boland. 1992. Posttranslational processing of gastrin in neoplastic human colonic tissues. Biochem Biophys Res Commun. 189:1165-9.
Koh, T.J., C.J. Bulitta, J.V. Fleming, G.J. Dockray, A. Varro, and T.C. Wang. 2000. Gastrin is a target of the beta-cateninlTCF-4 growth-signaling pathway in a model of intestinal polyposis. J Clin Invest. 106:533-9.
Koh, T.J., G.J. Dockray, A. Varro, R.J. Cahill, C.A. Dangler, J.G. Fox, and T.C. Wang. 1999. Overexpression of glycine-extended gastrin in transgenic mice results in increased colonic proliferation. J Clin Invest. 103:1119-26.
Konturek, P.C., W. Bielanski, S.J. Konturek, A. Hartwich, P. Pierzchalski, M. Gonciarz, K. Mariiez, T. Starzynska, M. Zuchowicz, Z. Darasz, J.P. Gotze, J.F. Rehfeld, and E.G. Hahn. 2002. Progastrin and cyclooxygenase-2 in colorectal cancer. Dig Dis Sci. 47:1984-91.
Korinek, V., N. Barker, P.J. Morin, D. van Wichen, R. de Weger, K.W. Kinzler, B. Vogelstein, and H. Clevers. 1997. Constitutive transcriptional activation by a beta-catenin-Tcf complex in APC-/- colon carcinoma. Science. 275:1784-7. Koyama, T., K. Tago, T. Nakamura, S. Ohwada, Y. Morishita, J. Yokota, and T. Akiyama. 2002. Mutation and expression of the beta-catenin-interacting protein ICAT in human colorectal tumors. Jpn J Clin Oncol. 32:358-62.
Lallemand-Breitenbach, V., J. Zhu, S. Kogan, Z. Chen, and H. de The. 2005. Opinion: how patients have benefited from mouse models of acute promyelocytic leukaemia. Nat Rev Cancer. 5:821-7*.*
Lian, Z., and A. Di Cristofano. 2005. Class reunion: PTEN joins the nuclear crew. Oncogene. 24:7394-400.
Litvak, D.A., M.R. Hellmich, K. Iwase, B.M. Evers, J. Martinez, M. Amblard, and C.M. Townsend, Jr. 1999. JMV1I55: a novel inhibitor of glycine-extended progastrin-mediated growth of a human colon cancer in vivo. Anticancer Res. 19:45-9.
Marotta, A., K. Parhar, D. Owen, S. Dedhar, and B. Salh. 2003. Characterisation of integrin-linked kinase signalling in sporadic human colon cancer. Br J Cancer. 88:1755-62.
Marques, J.T., T. Devosse, D. Wang, M. Zamanian-Daryoush, P. Serbinowski, R. Hartmann, T. Fujita, M.A. Behlke, and B.R. Williams. 2006. A structural basis for discriminating between self and nonself double-stranded RNAs in mammalian cells. Nat Biotechnol.
Morin, P.J., A.B. Sparks, V. Korinek, N. Barker, H. Clevers, B. Vogelstein, and K.W. Kinzler. 1997. Activation of beta-catenin-Tcf signaling in colon cancer by mutations in beta-catenin or APC. Science. 275:1787-90.
Mulrooney, J., K. Foley, S. Vineberg, M. Barreuther, and L. Grabel. 2000.Phosphorylation of the betal integrin cytoplasmic domain: toward an understanding of function and mechanism. Exp Cell Res. 258:332-41.
Nemeth, J., B. Taylor, S. Pauwels, A. Varro, and G.J. Dockray. 1993. Identification of progastrin derived peptides in colorectal carcinoma extracts. Gut. 34:90-5.
Nordlund MS, Fermer C, Nilsson O, Warren DJ, and E. Paus. 2007. Production and Characterization of Monoclonal Antibodies for Immunoassay of the Liang Cancer Marker proGRP. Tumour Biol. 28(2):100-10.
Ottewell, P.D., A. Varro, G.J. Dockray, C.M. Kirton, A.J. Watson, T.C. Wang, R. Dimaline, and D.M. Pritchard. 2005. COOH-terminal 26-amino acid residues of progastrin are sufficient for stimulation of mitosis in murine colonic epithelium in vivo. Am J Physiol Gastrointest Liver Physiol. 288:G541-9.
Ottewell, P.D., A.J. Watson, T.C. Wang, A. Varro, G.J. Dockray, and D.M. Pritchard. 2003. Progastrin stimulates murine colonic epithelial mitosis after DNA damage. Gastroenterology. 124:1348-57.
Pear, W.S., G.P. Nolan, M.L. Scott, and D. Baltimore. 1993. Production of high-titer helper-free retroviruses by transient transfection. Proc Nad Acad Sci USA. 90:8392-6.
Radtke, F., and H. Clevers. 2005. Self-renewal and cancer of the gut: two sides of a coin. Science. 307:1904-9.
Sekiya, T., T. Nakamura, Y. Kazuki, M. Oshimura, K. Kohu, K. Tago, S. Ohwada, and T. Akiyama. 2002. Overexpression of Icat induces G(2) arrest and cell death in tumor cell mutants for adenomatous polyposis coli, beta-catenin, or Axin. Cancer Res. 62:3322-6.
Seva, C., C.J. Dickinson, and T. Yamada. 1994. Growth-promoting effects of glycine-extended progastrin. Science. 265:410-2.
Siddheshwar, R.K., J.C. Gray, and S.B. Kelly. 2001. Plasma levels of progastrin but not amidated gastrin or glycine extended gastrin are elevated in patients with colorectal carcinoma. Gut. 48:47-52.
Singh, P., A. Owlia, A. Varro, B. Dai, S. Rajaraman, and T. Wood. 1996. Gastrin gene expression is required for the proliferation and tumorigenicity of human colon cancer cells. Cancer Res. 56:4111-5.
Singh, P., M. Velasco, R. Given, A. Varro, and T.C. Wang. 2000. Progastrin expression predisposes mice to colon carcinomas and adenomas in response to a chemical carcinogen. Gastroenterology. 119:162-71.
Singh, P., Z. Xu, B. Dai, S. Rajaraman, N. Rubin, and B. Dhruva. 1994. Incomplete processing of progastrin expressed by human colon cancer cells: role of noncarboxyamidated gastrins. Am J Physiol. 266:G459-68.
Stepan, V.M., M. Sawada, A. Todisco, and C.J. Dickinson. 1999. Glycine-extended gastrin exerts growth-promoting effects on human colon cancer cells. Mol Med. 5:147-59.
Tago, K., T. Nakamura, M. Nishita, J. Hyodo, S. Nagai, Y. Murata, S. Adachi, S. Ohwada, Y. Morishita, H. Shibuya, and T. Akiyama. 2000. Inhibition of Wnt signaling by ICAT, a novel beta-catenin-interacting protein. Genes Dev. 14:1741-9.
Tan, C., P. Costello, J. Sanghera, D. Dominguez, J. Baulida, A.G. de Herreros, and S. Dedhar. 2001. Inhibition of integrin linked kinase (ILK) suppresses beta-catenin-Lef/Tcf-dependent transcription and expression of the E-cadherin repressor, snail, in APC-/- human colon carcinoma cells. Oncogene. 20:133-40.
van de Wetering, M., E. Sancho, C. Verweij, W. de Lau, I. Oving, A. Hurlstone, K. van der Horn, E. Baffle, D. Coudreuse, A.P. Haramis, M. Tjon-Pon-Fong, P. Moerer, M. van den Born, G. Soete, S. Pals, M. Eilers, R. Medema, and H. Clevers. 2002. The beta-catenin/TCF-4 complex imposes a crypt progenitor phenotype on colorectal cancer cells. Cell. 111:241-50.
van Es, J.H., and H. Clevers. 2005. Notch and Wnt inhibitors as potential new drags for intestinal neoplastic disease. Trends Mol Med. 11:496-502.
Van Solinge, W.W., F.C. Nielsen, L. Friis-Hansen, U.G. Falkmer, and J.F. Rehfeld. 1993. Expression but incomplete maturation of progastrin in colorectal carcinomas. Gastroenterology. 104:1099-107.
Wang, T.C., T.J. Koh, A. Varro, R.J. Cahill, C.A. Dangler, J.G. Fox, and G.J. Dockray. 1996. Processing and proliferative effects of human progastrin in transgenic mice. J Clin Invest. 98:1918-29.
Wang, Z.Y., and Z. Chen. 2000. Differentiation and apoptosis induction therapy in acute promyelocytic leukaemia. Lancet Oncol. 1:101-6*.*
Wray C.J., Rilo H.L., and S.A. Ahmad. 2004. Colon cancer angiogenesis and antiangiogenic therapy. Expert Opin Investig Drugs. 13(6):631-41.

## Claims

1. An antibody or antibody binding fragment that specifically binds progastrin for use in treating colorectal cancer.

2. The antibody or antibody binding fragment for use of claim 1 in which the beta-catenin/Tcf-4-mediated transcriptional pathway of the colorectal cancer being treated is constitutively active.

3. The antibody or antibody binding fragment for use of claim 1 in which the cells of the colorectal cancer being treated contain a mutation in their adenomatous polyposis coli (APC) gene.

4. The antibody or antibody binding fragment for use of claim 1 in which the cells of the colorectal cancer being treated contain a mutation in their beta-catenin gene.

5. The antibody or antibody binding fragment for use according to any one of claims 1-4 which is selective for progastrin as compared to amidated or glycine-extended forms of gastrin 17 and gastrin 34.

6. The antibody or antibody binding fragment for use of claim 5 which binds a C-terminal region of human progastrin.

7. The antibody or antibody binding fragment for use of claim 5 which binds to FGRRSAEDEN (SEQ ID NO:31).

8. The antibody or antibody binding fragment for use of claim 5 which binds an N-terminal region of human progastrin.

9. The antibody or antibody binding fragment for use of claim 5 which binds to SWKPRSQQPDAPLGT (SEQ ID NO:32).

## Patentansprüche

1. Antikörper oder antikörperbindendes Fragment, der bzw. das spezifisch Progastrin bindet, zur Verwendung beim Behandeln eines kolorektalen Karzinoms.

2. Antikörper oder antikörperbindendes Fragment zur Verwendung nach Anspruch 1, wobei der beta-Catenin/Tcf-4-vermittelte Transkriptionsweg des behandelten kolorektalen Karzinoms konstitutiv aktiv ist.

3. Antikörper oder antikörperbindendes Fragment zur Verwendung nach Anspruch 1, wobei die Zellen des behandelten kolorektalen Karzinoms eine Mutation in ihrem Adenomatösen-Polyposis-coli-Gen (APC) enthalten.

4. Antikörper oder antikörperbindendes Fragment zur Verwendung nach Anspruch 1, wobei die Zellen des behandelten kolorektalen Karzinoms eine Mutation in ihrem beta-Catenin-Gen enthalten.

5. Antikörper oder antikörperbindendes Fragment zur Verwendung nach einem der Ansprüche 1 bis 4, der bzw. das selektiv für Progastrin im Vergleich zu amidierten oder glycinerweiterten Formen von Gastrin 17 und Gastrin 34 ist.

6. Antikörper oder antikörperbindendes Fragment zur Verwendung nach Anspruch 5, der bzw. das eine C-terminale Region von humanem Progastrin bindet.

7. Antikörper oder antikörperbindendes Fragment zur Verwendung nach Anspruch 5, der bzw. das FGRRSAEDEN (SEQ ID Nr.: 31) bindet.

8. Antikörper oder antikörperbindendes Fragment zur Verwendung nach Anspruch 5, der bzw. das eine N-terminale Region von humanem Progastrin bindet.

9. Antikörper oder antikörperbindendes Fragment zur Verwendung nach Anspruch 5, der bzw. das SWKPRSQQPDAPLGT (SEQ ID Nr.: 32) bindet.

## Revendications

1. Anticorps ou fragment de liaison d'un anticorps qui se lie spécifiquement à la progastrine pour une utilisation dans le traitement du cancer colorectal.

2. Anticorps ou fragment de liaison d'un anticorps pour une utilisation selon la revendication 1, dans laquelle la voie transcriptionnelle à médiation par la bêta-caténine/Tcf-4 du cancer colorectal qui est traité est constitutivement active.

3. Anticorps ou fragment de liaison d'un anticorps pour une utilisation selon la revendication 1, dans laquelle les cellules du cancer colorectal qui est traité contiennent une mutation dans leur gène adenomatous polyposis coli (APC).

4. Anticorps ou fragment de liaison d'un anticorps pour une utilisation selon la revendication 1, dans laquelle les cellules du cancer colorectal qui est traité contiennent une mutation dans leur gène de la bêtacaténine.

5. Anticorps ou fragment de liaison d'un anticorps pour une utilisation selon l'une quelconque des revendications 1 à 4, qui est sélectif pour la progastrine par rapport aux formes amidées ou à extension de glycine de la gastrine 17 et de la gastrine 34.

6. Anticorps ou fragment de liaison d'un anticorps pour une utilisation selon la revendication 5, qui se lie à une région C-terminale de la progastrine humaine.

7. Anticorps ou fragment de liaison d'un anticorps pour une utilisation selon la revendication 5, qui se lie à FGRRSAEDEN (SEQ ID N° : 31).

8. Anticorps ou fragment de liaison d'un anticorps pour une utilisation selon la revendication 5, qui se lie à une région N-terminale de la progastrine humaine.

9. Anticorps ou fragment de liaison d'un anticorps pour une utilisation selon la revendication 5, qui se lie à SWKPRSQQPDAPLGT (SEQ ID N° : 32).


### SEQUENCE LISTING

<110> INSERM
<120> Inhibitors of progastrin-induced repression of ICAT for treating and/or preventing colorectal cancer or adenomatous polyposis displaying progastrin-secreting colonic cells and colonic cells in which the beta-catenin/Tcf-4-mediated transcriptional pathway is constitutively active.
<130> BEP060186
<160> 40
<170> PatentIn version 3.3
<210> 1
   <211> 53
   <212> DNA
   <213> artificial
<220>
   <223> shRNA
<400> 1
   gaagaagcct atggatggat tcaagagaag gtaggtatcc gaagaagttt ttt 53
<210> 2
   <211> 61
   <212> DNA
   <213> artificial
<220>
   <223> shRNA
<400> 2
   aattaaaaaa cttcttcgga tacctacctt ctcttgaatc catccatagg cttcttcggc 60
   c 61
<210> 3
   <211> 67
   <212> DNA
   <213> artificial
<220>
   <223> shRNA
<400> 3
   gatcccaagg ccagacgcga attatttttc aagagaaaat aattcgcgtc tggccttttt 60
   ttggaaa 67
<210> 4
   <211> 67
   <212> DNA
   <213> artificial
<220>
   <223> shRNA
<400> 4
   agcttttcca aaaaaaggcc agacgcgaat tattttctct tgaaaaataa ttcgcgtctg 60
   gccttgg 67
<210> 5
   <211> 64
   <212> DNA
   <213> artificial
<220>
   <223> shRNA
<400> 5
   gatccggatg ggatcaaacc tgacattttc aagagaaatg tcaggtttga tcccatcttt 60
   tttg 64
<210> 6
   <211> 64
   <212> DNA
   <213> artificial
<220>
   <223> shRNA
<400> 6
   aattcaaaaa agatgggatc aaacctgaca tttctcttga aaatgtcagg tttgatccca 60
   tccg 64
<210> 7
   <211> 56
   <212> DNA
   <213> artificial
<220>
   <223> shRNA
<400> 7
   gatccgacat taagaagggc ccagcttttc aagagagctg ggccttaatc tttttt 56
<210> 8
   <211> 50
   <212> DNA
   <213> artificial
<220>
   <223> shRNA
<400> 8
   aattgattaa ggcccagctc tcttgaaaag ctgggccctt cttaatgtcg 50
<210> 9
   <211> 23
   <212> DNA
   <213> artificial
<220>
   <223> primer
<400> 9
   ggtggtctcc tctgacttca aca 23
<210> 10
   <211> 23
   <212> DNA
   <213> artificial
<220>
   <223> primer
<400> 10
   gttgctgtag ccaaattcgt tgt 23
<210> 11
   <211> 18
   <212> DNA
   <213> artificial
<220>
   <223> primer
<400> 11
   cgggaatctg cgtgacat 18
<210> 12
   <211> 20
   <212> DNA
   <213> artificial
<220>
   <223> primer
<400> 12
   aaggaaggct ggaagagtgc 20
<210> 13
   <211> 21
   <212> DNA
   <213> artificial
<220>
   <223> primer
<400> 13
   atgactgccc gaattagcat g 21
<210> 14
   <211> 20
   <212> DNA
   <213> artificial
<220>
   <223> primer
<400> 14
   gctacccagg caggtgcata 20
<210> 15
   <211> 20
   <212> DNA
   <213> artificial
<220>
   <223> primer
<400> 15
   gctctggtgc tttagttagg 20
<210> 16
   <211> 21
   <212> DNA
   <213> artificial
<220>
   <223> primer
<400> 16
   gcacttggtt tctttctttt c 21
<210> 17
   <211> 23
   <212> DNA
   <213> artificial
<220>
   <223> primer
<400> 17
   cgtctccaca catcagagca caa 23
<210> 18
   <211> 22
   <212> DNA
   <213> artificial
<220>
   <223> primer
<400> 18
   tcttggcagc aggatagtcc tt 22
<210> 19
   <211> 33
   <212> DNA
   <213> artificial
<220>
   <223> primer
<400> 19
   agaggatcca aatgcagcga ctatgtgtgt atg 33
<210> 20
   <211> 35
   <212> DNA
   <213> artificial
<220>
   <223> primer
<400> 20
   ggcgaattcc taggattgtt agttctcatc ctcag 35
<210> 21
   <211> 22
   <212> DNA
   <213> artificial
<220>
   <223> primer
<400> 21
   tgggtgtccc tcgtctccta ca 22
<210> 22
   <211> 19
   <212> DNA
   <213> artificial
<220>
   <223> primer
<400> 22
   tgttgccaaa ccggtggta 19
<210> 23
   <211> 20
   <212> DNA
   <213> artificial
<220>
   <223> primer
<400> 23
   ctccaacatg gacattgacg 20
<210> 24
   <211> 20
   <212> DNA
   <213> artificial
<220>
   <223> primer
<400> 24
   cagtgcggtt ccacacatac 20
<210> 25
   <211> 22
   <212> DNA
   <213> artificial
<220>
   <223> primer
<400> 25
   atcaccgcca ctgccaccac ca 22
<210> 26
   <211> 30
   <212> DNA
   <213> artificial
<220>
   <223> primer
<400> 26
   caccttagtg tccccttttg tcatagggct 30
<210> 27
   <211> 21
   <212> DNA
   <213> artificial
<220>
   <223> siRNA
<400> 27
   gaagaagccu auggauggat t 21
<210> 28
   <211> 21
   <212> DNA
   <213> artificial
<220>
   <223> siRNA
<400> 28
   uccauccaua ggcuucuuct t 21
<210> 29
   <211> 20
   <212> DNA
   <213> artificial
<220>
   <223> siRNA
<400> 29
   gaagaggccu acggauggtt 20
<210> 30
   <211> 20
   <212> DNA
   <213> artificial
<220>
   <223> siRNA
<400> 30
   ccauccguag gccucuuctt 20
<210> 31
   <211> 10
   <212> PRT
   <213> Artificial
<220>
   <223> epitope
<400> 31
   Phe Gly Arg Arg Ser Ala Glu Asp Glu Asn
   1               5                   10
<210> 32
   <211> 15
   <212> PRT
   <213> Artificial
<220>
   <223> epitope
<400> 32
   Ser Trp Lys Pro Arg Ser Gln Gln Pro Asp Ala Pro Leu Gly Thr
   1               5                   10                  15
<210> 33
   <211> 23
   <212> DNA
   <213> artificial
<220>
   <223> primer
<400> 33
   ggtggtctcc tctgacttca aca 23
<210> 34
   <211> 23
   <212> DNA
   <213> Artificial
<220>
   <223> primer
<400> 34
   gttgctgtag ccaaattcgt tgt 23
<210> 35
   <211> 20
   <212> DNA
   <213> Artificial
<220>
   <223> primer
<400> 35
   gctctggtgc tttagttagg 20
<210> 36
   <211> 21
   <212> DNA
   <213> Artificial
<220>
   <223> primer
<400> 36
   gcacttggtt tctttctttt c 21
<210> 37
   <211> 23
   <212> DNA
   <213> Artificial
<220>
   <223> primer
<400> 37
   cgtctccaca catcagagca caa 23
<210> 38
   <211> 22
   <212> DNA
   <213> Artificial
<220>
   <223> primer
<400> 38
   tcttggcagc aggatagtcc tt 22
<210> 39
   <211> 18
   <212> DNA
   <213> Artificial
<220>
   <223> primer
<400> 39
   ccgtccatgg ggaagatc 18
<210> 40
   <211> 17
   <212> DNA
   <213> Artificial
<220>
   <223> primer
<400> 40
   atggccagcg ggaagac 17